# EUROPÄISCHE PATENTSCHRIFT

(11) **EP 1 794 293 B1**
(45) Veröffentlichungstag und Bekanntmachung des Hinweises auf die Patenterteilung: **22.09.2010**
(21) Anmeldenummer: 05796115.3
(22) Anmeldetag: 22.09.2005
(51) Int. Cl.: C12N 9/28, C11D 3/386

(54) **ALPHA-AMYLASE-VARIANTEN MIT ERHÖHTER LÖSUNGSMITTELSTABILITÄT, VERFAHREN ZU DEREN HERSTELLUNG SOWIE WASCH- UND REINIGUNGSMITTEL MIT DIESEN ALPHA-AMYLASE-VARIANTEN**
ALPHA AMYLASE VARIANTS HAVING AN ELEVATED SOLVENT STABILITY, METHOD FOR THE PRODUCTION THEREOF AND DETERGENTS AND CLEANSERS CONTAINING THESE ALPHA AMYLASE VARIANTS
VARIANTES D'ALPHA-AMYLASE A STABILITE AMELIOREE VIS-A-VIS D'AGENTS SOLVANTS, PROCEDES PERMETTANT DE LES PRODUIRE ET DETERGENTS ET NETTOYANTS COMPRENANT LESDITES VARIANTES D'ALPHA-AMYLASE

(30) Priorität: 01.10.2004 DE 102004047777
(43) Veröffentlichungstag der Anmeldung: 13.06.2007
(73) Patentinhaber: Henkel AG & Co. KGaA, 40589 Düsseldorf (DE)
(72) Erfinder: BESSLER, Cornelius, 40597 Düsseldorf (DE); WIELAND, Susanne, 41541 Dormagen-Zons (DE); MAURER, Karl-Heinz, 40699 Erkrath (DE)
(86) Internationale Anmeldenummer: PCT/EP2005/010261
(87) Internationale Veröffentlichungsnummer: WO 2006/037484

(56) Entgegenhaltungen:
- WO-A-00/60059
- WO-A-00/60060
- WO-A-01/96537
- WO-A-02/10356
- WO-A-02/31124

### SEQUENCE LISTING

<110> Henkel Kommanditgesellschaft auf Aktien
<120> Alpha-Amylase-Varianten mit erhöhter Lösungsmittelstabilität, Verfahren zu deren Herstellung sowie Wasch- und Reinigungsmittel mit diesen Alpha-Amylase-Varianten
<130> H 06468 PCT
<150> DE102004047777.9
   <151> 2004-10-01
<160> 2
<170> PatentIn version 3.1
<210> 1
   <211> 1554
   <212> DNA
   <213> Bacillus sp. A 7-7 (DSM 12368)
<220>
   <221> gene
   <222> (1)..(1554)
   <223> Alpha-Amylase
<220>
   <221> CDS
   <222> (1)..(1551)
   <223>
<220>
   <221> mat_peptide
   <222> (100)..()
   <223>
<400> 1
   atg acg atg aga aaa cgt aaa aat gga tta atc agt att cta ttg gca       48
   Met Thr Met Arg Lys Arg Lys Asn Gly Leu Ile Ser Ile Leu Leu Ala
               -30                 -25                 -20
   ttt ttg ttg gta ctt aca tca ata cct ttt act tca gca aac gta gaa       96
   Phe Leu Leu Val Leu Thr Ser Ile Pro Phe Thr Ser Ala Asn Val Glu
           -15                 -10                 -5
   gca cac cat aat ggc aca aat gga aca atg atg caa tat ttt gaa tgg      144
   Ala His His Asn Gly Thr Asn Gly Thr Met Met Gln Tyr Phe Glu Trp
   -1  1               5                   10                  15
   tat ttg cca aat gac ggt aat cat tgg aat aga tta aga tca gat gca      192
   Tyr Leu Pro Asn Asp Gly Asn His Trp Asn Arg Leu Arg Ser Asp Ala
                   20                  25                  30
   agt aat ctt aaa gat aaa ggg att aca gcg gtt tgg ata cca cct gct      240
   Ser Asn Leu Lys Asp Lys Gly Ile Thr Ala Val Trp Ile Pro Pro Ala
               35                  40                  45
   tgg aaa ggg gct tct caa aat gat gta ggg tat gga gcc tat gat ctg      288
   Trp Lys Gly Ala Ser Gln Asn Asp Val Gly Tyr Gly Ala Tyr Asp Leu
           50                  55                  60
   tat gat tta gga gaa ttc aat caa aaa gga acc gta cgt aca aag tac      336
   Tyr Asp Leu Gly Glu Phe Asn Gln Lys Gly Thr Val Arg Thr Lys Tyr
       65                  70                  75
   gga acc cgt aat caa tta caa gct gca gta acc gcc tta aaa agt aat      384
   Gly Thr Arg Asn Gln Leu Gln Ala Ala Val Thr Ala Leu Lys Ser Asn
   80                  85                  90                  95
   ggt att caa gta tac gga gat gtc gta atg aat cat aag ggt gga gcg      432
   Gly Ile Gln Val Tyr Gly Asp Val Val Met Asn His Lys Gly Gly Ala
                   100                 105                 110
   gat gcc act gag tgg gtt cga gcg gtt gaa gtg aac cca agt aat cgt      480
   Asp Ala Thr Glu Trp Val Arg Ala Val Glu Val Asn Pro Ser Asn Arg
               115                 120                 125
   aat caa gaa gtc tct ggt gat tat acg att gag gct tgg act aag ttt      528
   Asn Gln Glu Val Ser Gly Asp Tyr Thr Ile Glu Ala Trp Thr Lys Phe
           130                 135                 140
   gat ttt cct ggt cga ggt aat acc cac tct aac ttt aaa tgg aga tgg      576
   Asp Phe Pro Gly Arg Gly Asn Thr His Ser Asn Phe Lys Trp Arg Trp
       145                 150                 155
   tat cat ttc gat ggt gta gat tgg gat cag tca cgt caa ttg cag aat      624
   Tyr His Phe Asp Gly Val Asp Trp Asp Gln Ser Arg Gln Leu Gln Asn
   160                 165                 170                 175
   cga atc tat aaa ttc aga gga gat gga aaa ggt tgg gac tgg gaa gtt      672
   Arg Ile Tyr Lys Phe Arg Gly Asp Gly Lys Gly Trp Asp Trp Glu Val
                   180                 185                 190
   gat aca gag aac gga aac tat gac tat cta atg tac gcg gat att gat      720
   Asp Thr Glu Asn Gly Asn Tyr Asp Tyr Leu Met Tyr Ala Asp Ile Asp
               195                 200                 205
   atg gat cac cct gaa gta gtg aat gaa ctc aga aac tgg ggt gta tgg      768
   Met Asp His Pro Glu Val Val Asn Glu Leu Arg Asn Trp Gly Val Trp
           210                 215                 220
   tat acc aat aca ctg ggg cta gac ggg ttc aga ata gat gcg gta aaa      816
   Tyr Thr Asn Thr Leu Gly Leu Asp Gly Phe Arg Ile Asp Ala Val Lys
       225                 230                 235
   cat ata aaa tat agc ttt act cgt gat tgg ctt act cac gtt aga aat      864
   His Ile Lys Tyr Ser Phe Thr Arg Asp Trp Leu Thr His Val Arg Asn
   240                 245                 250                 255
   acg aca ggt aaa aat atg ttt gca gtt gca gag ttc tgg aag aat gac      912
   Thr Thr Gly Lys Asn Met Phe Ala Val Ala Glu Phe Trp Lys Asn Asp
                   260                 265                 270
   ata ggt gca att gaa aat tac tta agt aaa aca aat tgg aat cat tca      960
   Ile Gly Ala Ile Glu Asn Tyr Leu Ser Lys Thr Asn Trp Asn His Ser
               275                 280                 285
   gtt ttt gat gtg ccc ctg cat tat aac ctt tat aat gca tcg aga agt     1008
   Val Phe Asp Val Pro Leu His Tyr Asn Leu Tyr Asn Ala Ser Arg Ser
           290                 295                 300
   ggt ggc aat tat gat atg agg caa ata ttt aat gga aca gtt gtt cag     1056
   Gly Gly Asn Tyr Asp Met Arg Gln Ile Phe Asn Gly Thr Val Val Gln
       305                 310                 315
   aga cat cct aca cat gct gta aca ttt gtt gat aac cat gat tca cag     1104
   Arg His Pro Thr His Ala Val Thr Phe Val Asp Asn His Asp Ser Gln
   320                 325                 330                 335
   ccg gaa gaa gcc cta gag tca ttt gtt gaa gag tgg ttc aaa ccg tta     1152
   Pro Glu Glu Ala Leu Glu Ser Phe Val Glu Glu Trp Phe Lys Pro Leu
                   340                 345                 350
   gcg tat gct ctc aca cta aca cgt gat caa gga tat cct tcc gtt ttt     1200
   Ala Tyr Ala Leu Thr Leu Thr Arg Asp Gln Gly Tyr Pro Ser Val Phe
               355                 360                 365
   tat gga gat tat tat ggg att ccg acg cat ggt gta cca gca atg aaa     1248
   Tyr Gly Asp Tyr Tyr Gly Ile Pro Thr His Gly Val Pro Ala Met Lys
           370                 375                 380
   tct aag att gat ccg att tta gaa gca cgt caa aag tat gcg tac gga     1296
   Ser Lys Ile Asp Pro Ile Leu Glu Ala Arg Gln Lys Tyr Ala Tyr Gly
       385                 390                 395
   aaa caa aat gat tat ttg gat cac cat aat atg att ggc tgg acg cgt     1344
   Lys Gln Asn Asp Tyr Leu Asp His His Asn Met Ile Gly Trp Thr Arg
   400                 405                 410                 415
   gaa ggt aat aca gca cat ccc aac tca gga cta gca act att atg tcg     1392
   Glu Gly Asn Thr Ala His Pro Asn Ser Gly Leu Ala Thr Ile Met Ser
                   420                 425                 430
   gat ggc cca gga gga aat aaa tgg atg tat gtt ggg cgt aat aag gct     1440
   Asp Gly Pro Gly Gly Asn Lys Trp Met Tyr Val Gly Arg Asn Lys Ala
               435                 440                 445
   gga caa gtt tgg aga gat att aca gga aat cgc tca ggt acg gtg acg     1488
   Gly Gln Val Trp Arg Asp Ile Thr Gly Asn Arg Ser Gly Thr Val Thr
           450                 455                 460
   att aac gca gat ggg tgg ggt aat ttt tct gta aat ggt ggg tct gta     1536
   Ile Asn Ala Asp Gly Trp Gly Asn Phe Ser Val Asn Gly Gly Ser Val
       465                 470                 475
   tct ata tgg gta aat aat                                             1554
   Ser Ile Trp Val Asn
   480
<210> 2
   <211> 517
   <212> PRT
   <213> Bacillus sp. A 7-7 (DSM 12368)
<400> 2
   Met Thr Met Arg Lys Arg Lys Asn Gly Leu Ile Ser Ile Leu Leu Ala
               -30                 -25                 -20
   Phe Leu Leu Val Leu Thr Ser Ile Pro Phe Thr Ser Ala Asn Val Glu
           -15                 -10                 -5
   Ala His His Asn Gly Thr Asn Gly Thr Met Met Gln Tyr Phe Glu Trp
   -1  1               5                   10                  15
   Tyr Leu Pro Asn Asp Gly Asn His Trp Asn Arg Leu Arg Ser Asp Ala
                   20                  25                  30
   Ser Asn Leu Lys Asp Lys Gly Ile Thr Ala Val Trp Ile Pro Pro Ala
               35                  40                  45
   Trp Lys Gly Ala Ser Gln Asn Asp Val Gly Tyr Gly Ala Tyr Asp Leu
           50                  55                  60
   Tyr Asp Leu Gly Glu Phe Asn Gln Lys Gly Thr Val Arg Thr Lys Tyr
       65                  70                  75
   Gly Thr Arg Asn Gln Leu Gln Ala Ala Val Thr Ala Leu Lys Ser Asn
   80                  85                  90                  95
   Gly Ile Gln Val Tyr Gly Asp Val Val Met Asn His Lys Gly Gly Ala
                   100                 105                 110
   Asp Ala Thr Glu Trp Val Arg Ala Val Glu Val Asn Pro Ser Asn Arg
               115                 120                 125
   Asn Gln Glu Val Ser Gly Asp Tyr Thr Ile Glu Ala Trp Thr Lys Phe
           130                 135                 140
   Asp Phe Pro Gly Arg Gly Asn Thr His Ser Asn Phe Lys Trp Arg Trp
       145                 150                 155
   Tyr His Phe Asp Gly Val Asp Trp Asp Gln Ser Arg Gln Leu Gln Asn
   160                 165                 170                 175
   Arg Ile Tyr Lys Phe Arg Gly Asp Gly Lys Gly Trp Asp Trp Glu Val
                   180                 185                 190
   Asp Thr Glu Asn Gly Asn Tyr Asp Tyr Leu Met Tyr Ala Asp Ile Asp
               195                 200                 205
   Met Asp His Pro Glu Val Val Asn Glu Leu Arg Asn Trp Gly Val Trp
           210                 215                 220
   Tyr Thr Asn Thr Leu Gly Leu Asp Gly Phe Arg Ile Asp Ala Val Lys
       225                 230                 235
   His Ile Lys Tyr Ser Phe Thr Arg Asp Trp Leu Thr His Val Arg Asn
   240                 245                 250                 255
   Thr Thr Gly Lys Asn Met Phe Ala Val Ala Glu Phe Trp Lys Asn Asp
                   260                 265                 270
   Ile Gly Ala Ile Glu Asn Tyr Leu Ser Lys Thr Asn Trp Asn His Ser
               275                 280                 285
   Val Phe Asp Val Pro Leu His Tyr Asn Leu Tyr Asn Ala Ser Arg Ser
           290                 295                 300
   Gly Gly Asn Tyr Asp Met Arg Gln Ile Phe Asn Gly Thr Val Val Gln
       305                 310                 315
   Arg His Pro Thr His Ala Val Thr Phe Val Asp Asn His Asp Ser Gln
   320                 325                 330                 335
   Pro Glu Glu Ala Leu Glu Ser Phe Val Glu Glu Trp Phe Lys Pro Leu
                   340                 345                 350
   Ala Tyr Ala Leu Thr Leu Thr Arg Asp Gln Gly Tyr Pro Ser Val Phe
               355                 360                 365
   Tyr Gly Asp Tyr Tyr Gly Ile Pro Thr His Gly Val Pro Ala Met Lys
           370                 375                 380
   Ser Lys Ile Asp Pro Ile Leu Glu Ala Arg Gln Lys Tyr Ala Tyr Gly
       385                 390                 395
   Lys Gln Asn Asp Tyr Leu Asp His His Asn Met Ile Gly Trp Thr Arg
   400                 405                 410                 415
   Glu Gly Asn Thr Ala His Pro Asn Ser Gly Leu Ala Thr Ile Met Ser
                   420                 425                 430
   Asp Gly Pro Gly Gly Asn Lys Trp Met Tyr Val Gly Arg Asn Lys Ala
               435                 440                 445
   Gly Gln Val Trp Arg Asp Ile Thr Gly Asn Arg Ser Gly Thr Val Thr
           450                 455                 460
   Ile Asn Ala Asp Gly Trp Gly Asn Phe Ser Val Asn Gly Gly Ser Val
       465                 470                 475
   Ser Ile Trp Val Asn
   480

## Beschreibung

Die vorliegende Erfindung betrifft α-Amylase-Varianten, die durch Punktmutagenese von Oberflächenaminosäuren gegenüber einer über das Lösungsmittel ausgeübten Hydrolyse, insbesondere bei erhöhten Temperaturen oder hohen pH-Werten stabilisiert sind, Verfahren zu deren Herstellung über Mutagenese von bestimmten Oberflächenaminosäureresten sowie Wasch- und Reinigungsmittel mit diesen α-Amylase-Varianten.

α-Amylasen (E.C. 3.2.1.1) hydrolysieren interne α-1,4-glycosidische Bindungen von Stärke und stärkeähnlichen Polymeren unter Bildung von Dextrinen und β-1,6-verzweigten Oligosacchariden. Sie gehören zu den technisch wichtigsten Enzymen überhaupt. So werden α-Amylasen beispielsweise bei der Herstellung von Glucosesirup, zur Behandlung von Rohmaterialien in der Textilherstellung, zur Herstellung von Klebstoffen oder zur Herstellung von zuckerhaltigen Lebensmitteln oder Lebensmittelbestandteilen eingesetzt. Ein anderes wichtiges Einsatzgebiet ist die als aktive Komponente in Wasch- und Reinigungsmitteln.

Weil Wasch- und Reinigungsmittel überwiegend alkalische pH-Werte aufweisen, werden hierfür insbesondere α-Amylasen eingesetzt, die im alkalischen Medium aktiv sind. Solche werden von Mikroorganismen, das heißt Pilzen oder Bakterien, vor allem denen der Gattungen *Aspergillus* und *Bacillus* produziert und sekretiert. Ausgehend von diesen natürlichen Enzymen steht inzwischen eine nahezu unüberschaubare Fülle von Varianten zur Verfügung, die über Mutagenese abgeleitet worden sind und je nach Einsatzgebiet spezifische Vorteile aufweisen.

Beispiele hierfür sind die α-Amylasen aus *Bacillus licheniformis,* aus *B. amyloliquefaciens* und aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl^{®} und von der Firma Genencor unter dem Namen Purastar^{®}ST erhältlich. Weiterentwicklungsprodukte dieser α-Amylase sind von der Firma Novozymes unter den Handelsnamen Duramyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von *B. amyloliquefaciens* wird von der Firma Novozymes unter dem Namen BAN^{®} vertrieben, und abgeleitete Varianten von der α-Amylase aus *B. stearothermophilus* unter den Namen BSG^{®} und Novamyl^{®}, ebenfalls von der Firma Novozymes.

Beispiele für α-Amylasen aus anderen Organismen sind die unter den Handelsnamen Fungamyl^{®} von der Firma Novozymes erhältlichen Weiterentwicklungen der α-Amylase aus *Aspergillus niger* und *A.* oryzae. Ein weiteres Handelsprodukt ist beispielsweise die Amylase-LT^{®}.

Ferner sei auf die in der Anmeldung WO 02/10356 A2 offenbarte α-Amylase aus *Bacillus sp.* A 7-7 (DSM 12368) und die in der Anmeldung WO 02/44350 A2 beschriebene Cyclodextrin-Glucanotransferase (CGTase) aus *B.* agaradherens (DSM 9948) hingewiesen. Zusätzlich werden beispielsweise in den Anmeldungen WO 03/002711 A2 und WO 03/054177 A2 Sequenzräume von α-Amylasen definiert, die prinzipiell alle für entsprechende Anwendungen geeignet sein könnten.

Punktmutationen zur Verbesserung der Aktivität dieser Enzyme im alkalischen Medium werden beispielsweise in der nicht vorveröffentlichten Anmeldung DE 10309803.8 beschrieben. Dieser Anmeldung zufolge eignen sich hierfür Aminosäureaustausche in den Positionen 13, 32, 194, 197, 203, 230, 297, 356, 406, 414 und/oder 474 gemäß der Zählung der nicht prozessierten α-Amylase aus *B. amyloliquefaciens. -* Das sind in der Zählung der unprozessierten α-Amylase aus *Bacillus sp.* A 7-7 (DSM 12368; WO 02/10356 A2) die Positionen L13, T36, W198, S201, 1208, A235, D302, D361, H408, K416 beziehungsweise N476, mit folgenden besonders wirkungsvollen Autauschen: 13P, 32A, 194R, 197P, 203L, 230V, 297D, 356D, 406R, 414S und 474Q.

Ein weiteres Beispiel zur Punktmutagenese an α-Amylasen ist die Anmeldung WO 00/22103 A1. Darin werden Polypeptide, unter anderem auch α-Amylase-Varianten mit mutagenisierten Oberflächenaminosäuren offenbart. Der Zweck dieser Mutagenese hat darin bestanden, die von diesen Molekülen ausgehende Immunogenizität und/oder Allergenizität zu senken.

Ebenso sind Fusionsprodukte von α-Amylasen für den Einsatz in Wasch- und Reinigungsmitteln beschrieben. So werden beispielsweise in der Anmeldung WO 96/23874 A1 Hybride aus den α-Amylasen aus *Bacillus licheniformis, B. amyloliquefaciens* und *B. stearothermophilus* offenbart. Derartige Hybrid-Amylasen können nach der Lehre dieser Anmeldung zur Ermittlung der dreidimensionalen Struktur dieser Amylasen hergestellt werden, um darüber für die enzymatische Aktivität wichtige Positionen zu erkennen. Diesbezügliche Weiterentwicklungen stellen die Anmeldungen WO 97/41213 A1 und WO 00/60059 A2 dar, aus welchen zahlreiche α-Amylase-Varianten hervorgehen, die jeweils hinsichtlich ihrer Leistung verbessert sind. Spezielle Hybrid-Amylasen aus *B. licheniformis* und *B. amyloliquefaciens* offenbart die Anmeldung WO 03/014358 A2.

Einen weiteren wichtigen Stand der Technik bilden die drei Patentanmeldungen WO 96/23873 A1, WO 00/60060 A2 und WO 01/66712 A2, welche die Grundlage für das Handelsprodukt Stainzyme^{®} der Fa. Novozymes darstellen. All die darin jeweils ausgeführten durch Punktmutagenese erhältlichen Varianten besitzen geänderte enzymatische Eigenschaften und werden somit für den Einsatz in Wasch- und Reinigungsmitteln beansprucht beziehungsweise beschrieben. WO 96/23873 A1 nennt zum Teil jeweils mehrere Punktmutationen in mehr als 30 verschiedenen Positionen in vier verschiedenen Wildtypamylasen. Sie sollen geänderte enzymatische Eigenschaften hinsichtlich der Thermostabilität, der Oxidationsstabilität und der Calciumabhängigkeit aufweisen. Darunter sind Punktmutationen in folgenden Positionen, die jeweils unter Bezug auf die α-Amylase aus *Bacillus sp.* NCIB 12512 angegeben sind: Ersatz oxidierbarer Aminosäuren in den Positionen M9, M10, M105, M202, M208, M261, M309, M382, M430 oder M440, vorzugsweise M91L; M10L; M105L; M202L,T,F,I,V; M208L; M261L; M309L; M382L; M430L und M440L; Deletionen von F180, R181, G182, T183, G184 und/oder K185; sowie zusätzlich die Austausche K269R; P260E; R124P; M105F,I,L,V; M208F,W,Y; L217I; V206I,L,F; Y243F; K108R; K179R; K239R; K242R; K269R; D163N; D188N; D192N; D199N; D205N; D207N; D209N; E190Q; E194Q oder N106D.

Die Anmeldung WO 00/60060 A2 benennt ebenfalls eine Vielzahl an möglichen Aminosäureaustauschen in 10 verschiedenen Positionen, und zwar anhand' von zwei sehr ähnlichen α-Amylasen aus zwei verschiedenen Mikroorganismen gleicher Zählung (die α-Amylasen AA349 und AA4560). Das sind folgende Sequenzvariationen: R181*, G182*, D183*, G184*; N195A,R,D,C,E,Q,G,H,I,L,K,M,F,P,S,T,W,Y,V; I206A,R,D,N,C,E,Q,G,H,L,K,M,F,P,S,T,W,Y,V; E212A,R,D,N,C,Q,G,H,I,L,K,M,F,P,S,T,W,Y,V; E216A,R,D,N,C,Q,G,H,I,L,K,M,F,P,S,T,W,Y,V; K269A,R,D,N,C,E,Q,G,H,I,L,M,F,P,S,T,W,Y,V; und/oder R181A,N,D,C,E,Q,G,H,I,L,K,M,F,P,S,T,W,Y,V. Auch diese Entwicklung steht vor dem Hintergrund, über Mutationen eine Leistungsverbesserung zu erreichen.

Es existiert ein sehr umfangreicher Stand der Technik zur Verbesserung der Stabilität von α-Amylasen gegenüber den verschiedensten Einflüssen, indem an den betreffenden Molekülen Punktmutationen vorgenommen werden. Dieser soll hier in einem angemessenen Umfang gewürdigt werden, wobei lediglich die auf die Enzyme ausgerichteten Schriften berücksichtigt werden und weitgehend auf diejenigen Dokumente verzichtet wird, in denen wiederum der Einsatz der betreffenden (zuvor beschriebenen) Enzyme in verschiedensten Technikgebieten dargestellt wird:
- Die Anmeldungen WO 91/00343 A2 und EP 409299 A2 beschreiben Varianten der α-Amylase aus *B. amyloliquefaciens* mit verringerter Stabilität mit Austauschen in zum Teil mehreren der Positionen 113, 114, 116, 123, 163, 164, 166, 238, 316, 322, 345, 349, 356, 386, 394 und 398, darunter als besonders vorteilhaft die, bei der R123 gegen andere Aminosäuren, insbesondere C ausgetauscht worden ist.
- Die Anmeldung WO 02/31124 A2 benennt zahlreiche durch Zufallsmutagenese erhaltene Punktmutationen an der α-Amylase aus *B. sp.* KSM-K38. Sie sollen die Eigenschaften des Moleküls ändern, wobei die jeweiligen Einflüsse nicht detailliert benannt werden. Es sind dies folgende Austausche (in der Zählung dieser Amylase), wobei auch mehrere nebeneinander auftreten können: G2P,A; M9I,L,F; H14Y; L15M,I,F,T; E16P; H26Y,Q,R,N; D27N,S,T; G48A,V,S,T; N49X; Q51X; A52X; D53E,Q,R; V54X; A58V,L,I,F; V73L,I,F; E84Q; G88X; D94X; N96Q; M103I,L,F; N104D; M/L107G,A,V,T,S,I,L,F; G108A; F111G,A,V,I,L,T; A114D,I,L,M,V,R; T125S; D128T,E; S130T,C; Y133F,H; W138F,Y; G140H,R,K,D,N; D142H,R,K,N; S144P; N148S; A1491; R156H,K,D,N; N161X; W165R; D166E; R168P; E171L,I,F; H173R,K,L; I173L; L174I,F; A178N,Q,R,K,H; N179G,A,T,S; T180N,Q,R,K,H; N181X; N183X; W184R,K; D187N,S,T; E188P,T,I,S; N190F; D194X; L197X; G198X; S199X; N200X; I201L,M,F,Y; D202X; F203L,I,F,M; S204X; H205X; E207Y,R; Q209V,L,I,F,M; E210X; E211Q; L212I,F; D214N,R,K,H; D221N; E222Q,T; D224N,Q; Y228F; L230I,F; I233A,V,L,F; K234N,Q; P237X; W239X; T241L,I,F,M; S242P,R; A252T; D253G,A,V,N; Q254K; D255N,Q,E,P; G260A; K264Q,S,T; D265N,Y; V267L,I,F,M; D275N,T; E276K; M277T,I,L,F; E280N,T,Q,S; M281H,I,L,F; V286X, vorzugsweise V286Y,L,I,F; Y290X; Y293H,F; S301G,A,D,K,E,R; R305A,K,Q,E,H,D,N; E314K,Q,R,S,T,H,N; A315K,R,S; I318L,M,F; T329S; E333Q; A340R,K,N,D,Q,E; D341P,T,S,Q,N; G356Q,E,S,T,A; S375P; A376S; K377L,I,F,M; M3801,L,F; E383P,Q; L3841,F; D386N,Q,R,K,I,L; Q389K,R; Y399A,D,H; W403X; D404N; I405L,F; V406I,L,F,A,D; N427X; H441K,N,D,Q,E; R442Q; Q444E,K,R; A445V; Q448A; H453R,K,Q,N; A454S,T,P; G472R, N479Q,K,R; Q480K,R. Experimentell werden jedoch lediglich folgende Varianten offenbart: E84Q, N96D, A315S, A445V, G464N, N121D und N390H; ferner wird auf die Möglichkeit hingewiesen, die Substitutionen T125S, S144P, I173L, D210E, N393H, V4081, R442Q, N444H, Q448A und G464S vorzunehmen. Konkrete Effekte werden nicht gezeigt.

Diesen Anmeldungen zur Punktmutagenese ist gemeinsam, daß auch der Stabilitätsgesichtspunkt unter dem Aspekt einer guten Leistung im entsprechenden Einsatzgebiet bewertet wird. Denn die während der Lagerung und dem Einsatz der α-Amylasen beispielsweise im Rahmen von Waschmittelrezepturen aufrechterhaltene Stabilität führt zu einer hohen beziehungsweise möglichst gleichbleibend hohen Leistung bei entsprechendem Einsatz. Es sind keine Dokumente darunter, die sich gezielt mit der Stabilitätserhöhung während der Herstellung, Aufreinigung und Konfektionierung, das heißt der Prozessierung der α-Amylasen befassen.

Ein bislang wenig beachteter Aspekt in der Enzymentwicklung besteht darin, die Moleküle an sich so zu stabilisieren, daß sie bereits während ihrer Aufarbeitung eine gegenüber dem Wildtypmolekül erhöhte Stabilität aufweisen. Eine vorteilhafte zusätzliche Wirkung davon dürfte darin bestehen, daß diese gesteigerte Stabilität auch dem später beabsichtigten Einsatz des betreffenden Enzyms zugute kommen sollte.

Die Notwendigkeit hierfür zeigt sich insbesondere bei α-Amylasen. Zumindest einige davon zeigen vor allem während des Produktions- und Aufarbeitungsprozesses eine ausgesprochene Instabilität, was sich makroskopisch in einem Aktivitätsverlust zeigt. Dies gilt insbesondere für Verrfahrensschritte, in denen sie in wäßriger Lösung sind, insbesondere bei erhöhter Temperatur und bei erhöhten (stärker alkalischen) pH-Werten. Dadurch gehen die betreffenden Aktivitäten bereits bei der Aufarbeitung verloren. Unter dem Aufarbeitungsprozeß sind alle Schritte der großtechnischen Herstellung zu verstehen, von der Isolierung des betreffenden Enzyms, insbesondere den für die biotechnologische Gewinnung üblichen Fermentationsmedien, über die folgenden Wasch- und Separationsschritte (beispielsweise durch Fällung) und die Konzentrierung bis hin zur Konfektionierung, etwa der Granulation. Der Aktivitätsverlust kann aber auch bei der Lagerung von α-Amylase-haltigen Mitteln oder während der Anwendung auftreten, etwa bei Einsatz als aktiver Inhaltsstoff in Wasch- oder Reinigungsprozessen.

Diese Problematik kann soweit führen, daß sich einzelne α-Amylasen zwar im Labormaßstab gewinnen und untersuchen lassen, sich jedoch einer großtechnischen Herstellung mithilfe allgemein üblicher Methoden widersetzen. Man spricht dann auch davon, daß diese Enzyme eine niedrige Prozeßstabilität oder Prozessivität besitzen, womit die verschiedensten Verarbeitungs- und Einsatzmöglichkeiten gemeint sind. Beispielsweise die α-Amylase aus *Bacillus sp.* A 7-7 (DSM 12368) zeigt eine höhere Instabilität als die native α-Amylase aus *B. licheniformis.* Ansätze zur Behebung der Instabilität würden derartige Enzyme einer großtechnischen Produktion und damit den vielfältigen Einsatzgebieten überhaupt erst in technisch relevanten Mengen zugänglich machen.

Es stellte sich somit die Aufgabe, α-Amylasen aus *Bacillus sp.* A 7-7 (DSM 12368) an sich so zu stabilisieren, daß sie eine im Vergleich zum Ausgangsmolekül erhöhte Stabilität gegenüber einem Aktivitätsverlust, insbesondere bei erhöhter Temperatur und bei erhöhten (stärker alkalischen) pH-Werten aufweisen.

Unter einem Teilaspekt dieser Aufgabe mußte zunächst eine mögliche, in der Struktur der betreffenden Enzyme begründete Ursache für diese Anfälligkeit ermittelt werden. Anschließend galt es, dieser mit geeigneten Strukturveränderungen entgegenzutreten.

Diese Strukturveränderungen würden sich zum einen vorteilhaft auf die Aufarbeitung, das heißt die großtechnische Gewinnung dieser Enzyme auswirken. Zum anderen sollten sie auch für den Einsatz der α-Amylasen, etwa in Wasch- und Reinigungsmitteln vorteilhaft sein, weil damit zusätzlich eine gleichbleibend hohe Aktivität während des Einsatzes einhergehen sollte.

Als besonders vorteilhafte Lösungen dieser Aufgabe wären somit solche α-Amylasen anzusehen, die neben der genannten Stabilität weitere positive Eigenschaften hinsichtlich ihres beabsichtigten Einsatzes, insbesondere hinsichtlich ihres Einsatzes in Wasch- und Reinigungsmitteln zeigen.

Dies gilt vor allem für die α-Amylase aus *Bacillus sp.* A 7-7 (DSM 12368).

Als Grund für das insbesondere bei α-Amylasen, und ganz besonders bei bestimmten α-Amylasen beobachtete Phänomen des Aktivitätsverlusts in Lösung, auch in wäßriger Lösung und hierunter insbesondere bei alkalischen pH-Werten, und des Aktivitätsverlusts bei erhöhter Temperatur wurde auf dem Weg zur Lösung der genannten Aufgabe das Vorhandensein von besonders hydrolyseempfindlichen Aminosäureresten auf der Oberfläche dieser Moleküle in ihrer nativen, korrekt gefalteten globulären Struktur in Erwägung gezogen. Unter diesen stellten sich nicht die ionisierbaren basischen oder sauren Aminosäureseitenkeiten (H, K, R beziehungsweise D und E), nicht die mit einer Hydroxylgruppe (S und T), nicht die raumfüllenden zyklischen oder aromatischen (H beziehungsweise F, Y, W), nicht die schwefelhaltigen (C und M) oder die aliphatischen Aminaräureseitenketten (G, A, V, L und I) als wirkungsvollste Ansatzpunkte heraus, sondern die neutralen, säureamidgruppenhaltigen, das heißt Asparagin und Glutamin (N beziehungsweise Q). Diese scheinen insbesondere bei höheren Temperaturen und unter alkalischen Bedingungen hydrolysiert zu werden. Diese Hydrolyse dieser Reste scheint sich sodann destabilisierend auf die Struktur und damit die Funktionalität des gesamten Enzyms auszuwirken.

Zur Lösung der gestellten Aufgabe wird ein molekularbiologischer Ansatz verfolgt. Denn hierdurch wird auf die durch die bloße Aminosäuresequenz vermittellen enzymatischen Eigenschaften Einfluß genommen. Dabei wurde überraschenderweise festgestellt, daß Modifikationen, das heißt Punktmutationen, mit denen die auf der Oberfläche dieser α-Amylasen gelegenen Aminosäuren N und Q gegen andere Aminosäuren ausgetauscht werden, auf α-Amylasen stabilisierend wirken.

Welche Aminosäurereste "auf der Oberfläche dieser Moleküle" liegen, kann über die 3D-Struktur des globulären Enzymproteins, über die sogenannte Conolly-Oberfläche oder über den Wert der sogenannten Akzessibilität, das heißt der Lösungsmittelzugänglichkeit (siehe unten) definiert werden.

Ohne an diese Theorie gebunden sein zu wollen, kann man annehmen, daß die Substitution einer oder mehrerer auf Oberfläche von α-Amylasen gelegenen N- oder Q-Reste gegen andere Aminosäurereste die Molekülstruktur weniger beeinflußt, als wenn der betreffende Rest bei Lösung des Enzyms in einem wäßrigen, insbesondere alkalischen Lösungsmittel und/oder bei höheren Temperaturen unkontrolliert hydrolysiert wird. Dadurch liegen die Enzyme statistisch betrachtet vermehrt in ihrer nativen Konformation vor, wodurch die betreffende Präparation auch bei längerer Lagerung unter den genannten Bedingungen signifikant höhere Aktivitätswerte aufweist. Dies kommt sowohl der Gewinnung (etwa bei einem Fällungsschritt) und der Lagerung (etwa in Lösungsmitteln) als auch dem Einsatz zugute.

Diese Annahme wird dadurch folgenden Vergleich untermauert: Die α-Amylase aus *B. licheniformis* besitzt 18 N- und 14 Q-Reste (zusammen also 32) mit einer Akzessibilität von mindestens 10% auf seiner Oberfläche. Die aus *B. amyloliquefaciens* besitzt 22 N- und 19 solcher Q-Reste (zusammen 41), während es bei der α-Amylase aus *Bacillus sp.* A 7-7 (DSM 12368), wie in Beispiel 2 der vorliegenden Anmeldung vor Augen geführt wird, 41 N- und 14 derartiger Q-Reste (insgesamt also 55) sind. Der Lehre der vorliegenden Anmeldung zufolge bieten sich hier entsprechende Verbesserungsmöglichkeiten.

Die gestellte Aufgabe wird demzufolge durch α-Amylase-Varianten gemäß Anspruch 1 gelöst.

Die Aminosäuren werden in der vorliegenden Anmeldung mit dem international gebräuchlichen Ein- oder Dreibuchstabencode abgekürzt.

Unter α-Amylasen im Sinne der vorliegenden Anmeldung sind wie eingangs erläutert die Enzyme der Klasse E.C. 3.2.1.1 zu verstehen, die interne α-1,4-glycosidische Bindungen von Stärke und stärkeähnlichen Polymeren unter Bildung von Dextrinen und β-1,6-verzweigten Oligosaccharide hydrolysieren.

α-Amylase-Varianten sind solche α-Amylasen, die durch an sich bekannte gentechnische Modifizierungen aus einem Vorgängermolekül abgleitet worden sind. "Variante" ist auf der Ebene der Proteine der zu "Mutante" entsprechende Begriff auf der Ebene der Nukleinsäuren. Bei den Vorgänger- oder Ausgangsmolekülen kann es sich um Wildtypenzyme handeln, das heißt solche, die aus natürlichen Quellen erhältlich sind. Solche sind einleitend beispielhaft vorgestellt worden. Es kann sich auch um Enzyme handeln, die an sich bereits Varianten darstellen, das heißt gegenüber den Wildtypmolekülen bereits verändert worden sind. Darunter sind beispielsweise Punktmutanten, solche mit Änderungen der Aminosäuresequenz, über mehrere Positionen oder längere zusammenhängende Bereiche, oder auch Hybridmoleküle zu verstehen, die aus einander ergänzenden Abschnitten verschiedener Wildlyp-α-Amylasen zusammengesetzt sind. Sowohl Wildtypenzyme als auch Mutanten und Hybridenzyme sind einleitend beispielhaft vorgestellt worden. Sofern die für sie codierenden Nukleinsäuren bekannt sind, geschieht dies über etablierte Mutageneseverfahren an den betreffenden Nukleinsäuren; sollten sie nicht bekannt sein, können anhand der Aminosäuresequenz hierfür codierende Nukleinsäuresequenzen hergeleitet und entsprechend verändert werden.

Unter Aminosäureaustauschen sind Substitutionen einer Aminosäure gegen eine andere Aminosäure zu verstehen. Erfindungsgemäß werden solche Substitutionen unter Bezeichnung der Positionen, in der der Austausch erfolgt, gegebenenfalls kombiniert mit den betreffenden Aminosäuren im international gebräuchlichen Einbuchstabencode angegeben. "Austausch in Position 83" bedeutet beispielsweise, daß eine Variante in der Position, die in der Sequenz eines Referenzproteins die Position 83 aufweist, eine andere Aminosäure aufweist. Üblicherweise werden solche Austausche auf der DNA-Ebene über Mutationen einzelner Basenpaare durchgeführt (siehe oben). "N83D" bedeutet beispielsweise, daß das Referenzenzym an der Position 83 die Aminosäure Asparagin aufweist, während die betrachtete Variante an der hiermit homologisierbaren Position über die Aminosäure Asparaginsäure verfügt. "83D" bedeutet, daß jede beliebige, das heißt in der Regel eine natürlicherweise vorgegebene Aminosäure an einer Position, die der Position 83 entspricht, gegen eine Asparaginsäure ersetzt ist; und "N83X" bedeutet, daß die Aminosäure Asparagin in Position 83 gegen eine prinzipiell beliebige andere Aminosäure ersetzt ist.

Grundsätzlich sind die mit der vorliegenden Anmeldung bezeichneten erfindungsgemäßen Aminosäureaustausche nicht darauf beschränkt, daß sie die einzigen Austausche sind, in denen sich die betreffende Variante von dem Wildtypmolekül unterscheidet. Es ist im Stand der Technik bekannt, daß sich die vorteilhaften Eigenschaften einzelner Punktmutationen einander ergänzen können.

Als Referenzenzym hinsichtlich der Nummerierung der Positionen ist erfindungsgemäß die α-Amylase aus *Bacillus sp.* A 7-7 (DSM 12368) anzusehen, deren Nukleotidsequenz in SEQ ID NO. 1 angegeben ist, gefolgt von der zugehörigen Aminosäuresequenz in SEQ ID NO. 2. Diese Sequenzangaben sind, wie in Beispiel 1 der vorliegenden Anmeldung erläutert wird, gegenüber der Beschreibung in WO 02/10356 A2 in jeweils zwei Positionen korrigiert und entsprechen in dieser Form nach dem derzeitigen Kenntnisstand exakt den aus dem hinterlegten und in WO 02/10356 A2 beschriebenen Stamm DSM 12368 erhältlichen Sequenzdaten.

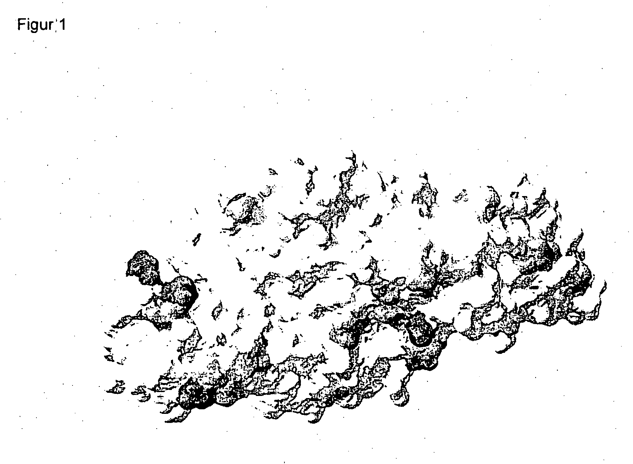

Diese korrekte Aminosäuresequenz geht auch aus der jeweils ersten Zeile des in Figur 1 gezeigten Alignments hervor, welches lediglich für die maturen Teile der jeweiligen Enzyme erstellt worden ist. Das ist dadurch gerechtfertigt, daß *in vivo* allein der mature Teil (das heißt der mit der positiven Zählung in SEQ ID NO. 2) als α-Amylase wirksam wird. Für manche Enzyme wie beispielsweise AA349 und AA560 werden in der Patentliteratur (WO 00/60060 A2) ohnehin lediglich die maturen Sequenzteile angegeben.

Die Oberfläche des Enzyms umfaßt all diejenigen Aminosäuren des nativ gefalteten Enzyms, die dem Lösungsmittel zugewandt sind. Das sind beispielsweise in der α-Amylase aus *Bacillus sp.* A 7-7 (DSM 12368) die 407 Aminosäuren, die im einzelnen in Beispiel 2 aufgeführt werden.

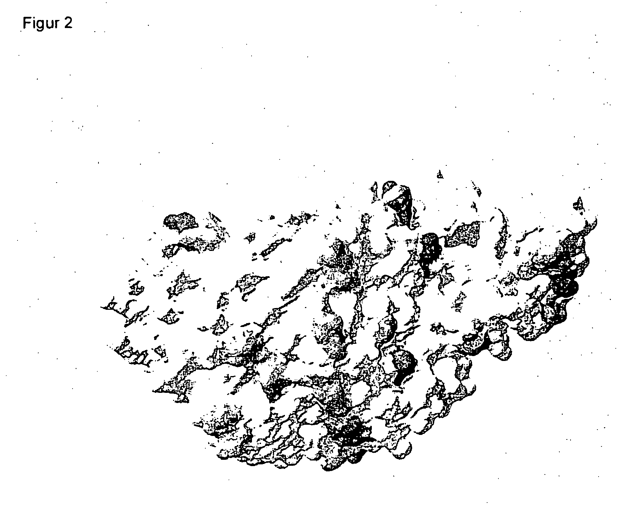

Die Oberflächenaminosäuren anderer α-Amylasen können prinzipiell unter Heranziehen des Alignments der Figur 2 erschlossen werden. Dies gilt jedoch nur näherungsweise. In hochkonservierten und strukturell gesicherten Bereichen wie α-Helices oder β-Faltblättern ergibt sich in der Regel eine gute Übereinstimmung; in flexibleren Bereichen, insbesondere Loops ist der auf dem Alignment, das heißt der Primärstruktur beruhende Vergleich mit Unsicherheiten behaftet. Ausschlaggebend ist in allen Fällen (sofern bekannt) die gesicherte Röntgenstruktur, wie sie tatsächlich für die meisten kommerziell wichtigen α-Amylasen inzwischen in allgemein zugänglichen Datenbanken hinterlegt ist. Das sind beispielsweise die Datenbanken GenBank (National Center for Biotechnology Information NCBI, National Institutes of Health, Bethesda, MD, USA) und Swiss-Prot (Geneva Bioinformatics (GeneBio) S.A., Genf, Schweiz; http://www.genebio.com/sprot.html).

Sollte für ein interessierendes Molekül die 3D- (oder Tertiär-)Struktur noch nicht bestimmt worden sein, so kann diese über ein Homologiemodelling erschlossen werden. Bei dieser Methode zur Strukturvorhersage von Proteinen, von denen noch keine Kristallstrukturen gelöst wurden, wird davon ausgegangen, daß Proteine mit ähnlicher Primärstruktur auch eine ähnliche Sekundär- und Tertiärstruktur besitzen. Die Ähnlichkeit zwischen zwei Proteinsequenzen läßt sich über einen geeigneten Algorithmus bestimmen, beispielsweise BLAST, FASTA oder CLUSTAL. Solche .Algorithmen stehen über allgemein zugängliche Protein-Datenbanken ebenfalls zur Verfügung; so verfügen beispielsweise GenBank und Swiss-Prot über entsprechende Verknüpfungen. Die RSCB-Protein-Databank (zugänglich über das Max-Delbrück-Zentrum in Berlin, Deutschland, über die Internet-Adresse http://www.pdb.mdc-berlin.de/pdb; 24.8.2004) bietet dem Benutzer die Möglichkeit, über eine FASTA-Suche zu einer bestimmten Sequenz Kristallstrukturen verwandter Proteine zu finden.

Die darauf aufbauenden, beispielsweise mithilfe des sogenannten Swiss-Pdb-Viewer möglichen Berechnungen werden in der Publikation "SWISS-Model and the Swiss-PdbViewer: An environment for comparative protein modeling" (1997) von N. Guex und M. C. Peitsch in Electrophoresis, Band 18, Seiten 2714 bis 2723, beschrieben. Dieser Viewer ist über die Internetadresse http://us.expasy.org/spdbv/ (24.8.2004) der Organisation Swiss Institute of Bioinformatics (Central Administration, Bätiment Ecole de Pharmacieroom 3041, Université de Lausanne, 1015 Lausanne, Switzerland) kostenlos zugänglich. Das zugehörige Handbuch (SwissPDB-Viewer-Manual) steht unter Internetadresse http://us.expasy.org/spdbv/text/selmenu.htm (24.8.2004) zur Verfügung. Durch Übereinanderlagerung (Superimposition) dieser Strukturen kann darüber eine Leitstruktur erstellt werden, auf die dann die Proteinsequenz der interessierenden α-Amylase aufmodelliert wird. Die einzelnen Schritte hierfür sind dem genannten Benutzerhandbuch zu entnehmen.

Allen hier diskutierten Darstellungen der Oberfläche eines Enzyms, das heißt der speziellen Aufzählung der Oberflächenaminosäuren der α-Amylase aus *Bacillus sp.* A 7-7 (DSM 12368; vergleiche Figur 1) als auch den dargestellten Modelling-Ansätzen liegt eine Berechnung zugrunde. Dabei wird kalkulatorisch eine Sonde der Größe von 1,4 Å über die Oberfläche des Proteins gerollt. Alle Positionen, die davon berührt werden, werden zur Kontaktfläche mit dieser Sonde und damit zur Oberfläche gezählt; engere, theoretisch nach außen offene Spalten werden dabei nicht mehr als Oberfläche angesehen sondern dem Molekülinneren zugerechnet. Nach diesem Ansatz erhält man die sogenannte Conolly-Oberfläche, die erstmals von M.L. Connolly in dem Aufsatz "Solvent-Accessible Surfaces of Proteins and Nucleic Acids" (1983) in Science, Band 221, 709-713, beschrieben worden ist. Diese anerkannte Methode ist dem Fachmann vertraut und dient erfindungsgemäß zur Definition der Oberfläche der α-Amylasen.

Für die α-Amylase aus *Bacillus sp.* A 7-7 (DSM 12368) ergaben sich auf diese Weise die erwähnten 407 Oberflächenaminosäuren, die im einzelnen in Beispiel 2 aufgeführt werden.

Hierunter befinden sich, wie weiterhin in Beispiel 2 dargestellt ist, für die α-Amylase aus *Bacillus sp.* A 7-7 (DSM 12368) als Teilmenge der zuvor bestimmten 407 Oberflächenaminosäurereste folgende 44 Asparaginreste:
N6 (12), N19 (28), N22 (28), N25 (16), N33 (28), N54 (35), N70 (38), N83 (44), N95 (19), N106 (1), N123 (19), N126 (18), N128 (31), N150 (39), N154 (41), N175 (20), N195 (24), N197 (3), N215 (27), N219 (32), N226 (40), N255 (42), N260 (49), N270 (38), N277 (24), N283 (40), N285 (27), N296 (15), N299 (23), N306 (51), N314 (35), N331 (6), N402 (16), N409 (11), N418 (25), N423 (40), N437 (37), N445 (41), N457 (29), N465 (22), N471 (20), N475 (25), N484 (12), N485 (38);
und folgende 17 Glutaminreste:
Q11 (4), Q53 (12), Q71 (5), Q84 (24), Q86 (18), Q98 (29), Q129 (31), Q169 (26), Q172 (53), Q174 (44), Q311 (35), Q319 (38), Q335 (3), Q361 (36), Q394 (20), Q401 (14), Q449 (31).

Diese N- und Q-Reste sind für die α-Amylase aus *Bacillus sp.* A 7-7 (DSM 12368) in den Darstellungen der Figuren 1 und 2 gezeigt. Man erkennt, daß sie in ungleichmäßiger Anordnung über die gesamte Moleküloberfläche verteilt sind.

Diese können erfindungsgemäß gegen prinzipiell alle anderen Aminosäurereste ausgetauscht werden, sofern es sich dabei nicht um N, Q, D oder E handelt. Daß eine wechselseitige Umwandlung von N zu Q beziehungsweise umgekehrt nicht sinnvoll ist, liegt auf der Hand. Der Ausschluß von D und E ist darauf begründet, daß auch diese, den Säureamidresten N und Q chemisch entsprechenden freien Säuregruppierungen ebenfalls hydrolyseanfällig sind. Erfindungsgemäß ist es deshalb empfehlenswert, Austausche gegen die verbleibenden natürlichen Aminosäuren vorzunehmen, das heißt zu A, C, F, G, H, I, K, L, M, P, R, S, T, V, W oder Y.

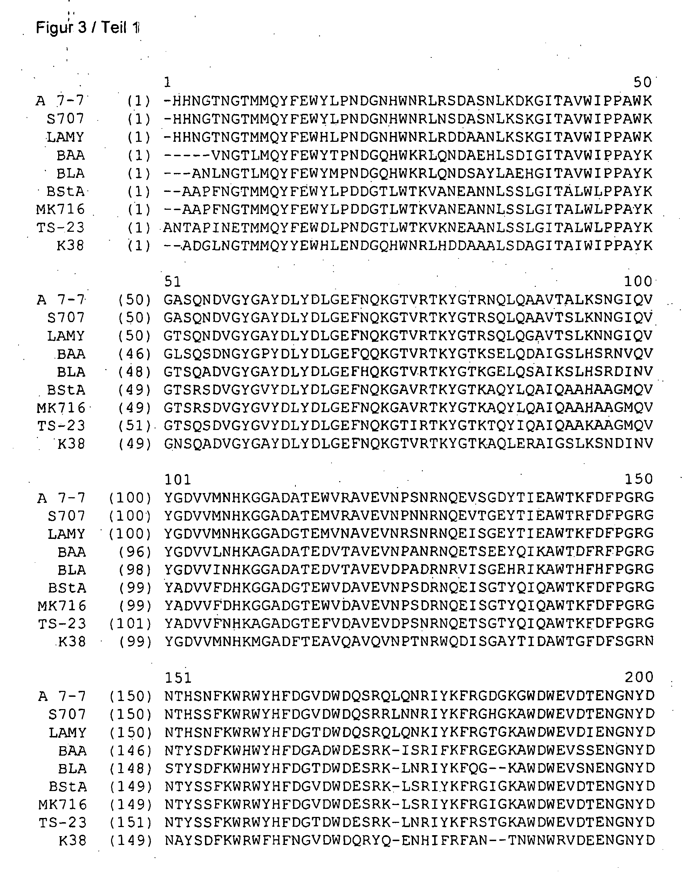

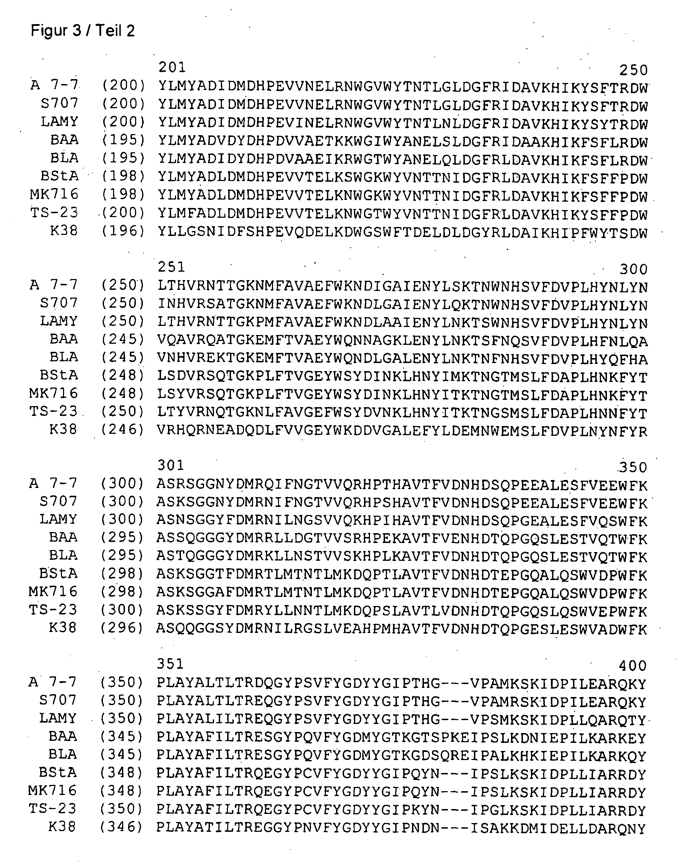

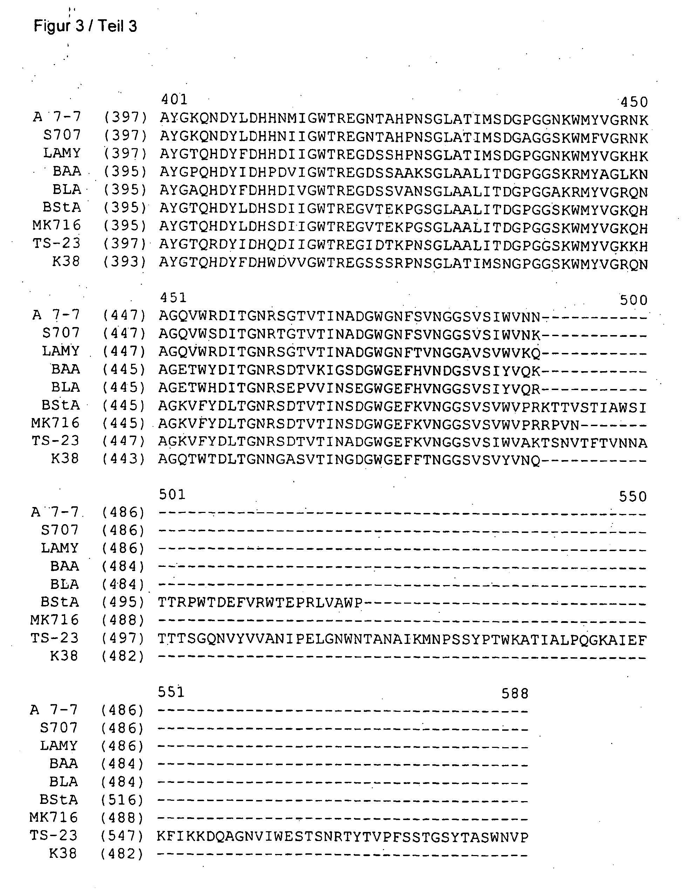

Zur Orientierung, welcher Austausch jeweils gewählt werden kann, sei - ohne hiermit eine Einschränkung der nachfolgend als bevorzugt bezeichneten Austausche vornehmen zu wollen - auf das Alignment der Figur 3 verwiesen. In zahlreichen dieser anhand der α-Amylase aus *Bacillus sp.* A 7-7 (DSM 12368) ermittelten Positionen liegen auch in anderen α-Amylasen N- oder Q-Reste. Biochemisch zumeist sinnvolle Alternativen hierzu gehen aus den Aminosäuresequenzen der anderen α-Amylasen hervor, die hier nicht über N oder Q verfügen. So ist auffällig, daß nirgends an homologer Position zu einem Oberflächen-Asparagin oder -Glutamin ein Methionin liegt. Sehr selten findet man dort ein C und nur etwas häufiger langkettige aliphatische Aminosäuren. Zur Entscheidung darüber, welche Aminosäure vorteilhafterweise an die Stelle eines N oder Q gesetzt werden kann, ist es ratsam, sich an diesem Alignment zu orientieren, wonach man anstelle dieser seltenen Reste zumeist einem kurzkettigen und/oder polaren Rest den Vorzug geben sollte.

Der Erfolg dieser erfindungsgemäßen Aminosäuresubstitution läßt sich, wie in Beispiel 4 erläutert ist, über die Solvens-Stabilität der betreffenden α-Amylase-Varianten überprüfen. Diese besitzen im Vergleich zu den jeweiligen Ausgangsenzymen, das heißt den Wildtypenzymen beziehungsweise denen, die gerade nicht die erfindungsgemäßen Austausche aufweisen, nach einer gewissen Zeit der Inkubation in einem entsprechenden Medium signifikant bessere Aktivitätswerte. Ein geeigneter Aktivitätstest ist in der Anmeldung WO 02/10356 A2 beschrieben. Besonders aussagekräftig ist dieser Vergleich, wenn bei der Inkubation vergleichsweise ungünstige Rahmenbedingungen herrschen, beispielsweise ein pH-Wert, der höher als das pH-Stabilitätsoptimum liegt, oder eine Temperatur, die über dem Temperaturoptimum der nicht erfindungsgemäßen Variante liegt. Denn dies sind, wie erläutert, die Situationen, gegenüber denen das betreffende Molekül besonders stabilisiert werden sollte. Erhöhte Aktivitätswerte sind dabei statistisch gesehen auf eine höhere Zahl von α-Amylase-Molekülen zurückzuführen, die die ungünstigigen Bedingungen unbeschadet überstanden haben.

In einer bevorzugten Ausführungsform handelt es sich bei erfindungsgemäßen α-Amylase-Varianten um solche, wobei das N durch ein A, G, K, R, S oder T, vorzugsweise durch ein S oder T beziehungsweise das Q durch ein A, G, I, K, R, S oder T, vorzugsweise durch ein S oder T ersetzt worden ist.

Unter den 16 noch verbleibenden Aminosäuren wird zur Substitution vorzugsweise nicht auf die raumfüllenden aliphatischen, schwefelhaltigen, aromatischen oder zyklischen Aminosäuren zurückgegriffen, weil diese unerwünschte hydrophobe Effekte hervorrufen könnten oder zusätzliche chemisch aktive Gruppen einbringen würden. Ferner wird vorzugsweise nicht auf P zurückgegriffen, weil dieses in der Regel mit erheblichen Strukturveränderungen verbunden ist. Für N bleiben vorzugsweise somit noch die Aminosäuren mit kleinen aliphatischen Gruppen, d. h. die neutralen Aminosäuren A und G, die zumeist geladenen, basischen Aminosäuren K und R sowie die polaren Reste von S und T zur Umsetzung der vorliegenden Erfindung übrig. Für Q gilt das gleiche, wobei sich der Austausch gegen I ebenfalls noch als vorteilhaft herausgestellt hat.

Im Einzelfall ist es empfehlenswert, unter diesen Möglichkeiten die jeweils am besten geeignete auszuwählen. In den meisten Fällen hat es sich als vorteilhaft herausgestellt, anstelle von N beziehungsweise Q eine der beiden Aminosäuren S oder T einzuführen. Ohne an diese Theorie gebunden sein zu wollen, kann man vermuten, daß dies an den nicht allzu stark voneinander abweichenden chemischen Eigenschaften dieser Reste liegt. Bei S und bei T handelt es sich wie bei N und Q um neutrale Seitenketten mit einem mittleren Raumbedarf; letzteres dürfte für die korrekte Faltung des Enzyms wichtig sein. Zudem sind beide Seitenketten polar, wobei die Werte für S und T mit +5,1 beziehungsweise +4,9 niedriger, aber noch im selben Größenbereich wie die für N und Q mit +9,7 beziehungsweise + 9,4 liegen. Dies ist vor allem vor dem Hintergrund wichtig, daß es sich um Oberflächenaminosäuren handelt, die nicht ins hydrophobe, apolare Molekülinnere ragen sondern mit der überwiegend hydrophilen, polaren Umgebung in Kontakt stehen.

In einer bevorzugten Ausführungsform handelt es sich bei erfindungsgemäßen α-Amylase-Varianten um solche, in denen der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% (nicht dem Solvens zugänglich) bis 100% (in einem hypothetischen Pentapeptid GGXGG enthalten) berechnet wird. Hierbei sind alle ganzzahligen und gebrochenen Zwischenwerte entsprechend eingeschlossen; zunehmend bevorzugte Ausführungsformen sind durch zunehmende Akzessibilitätswerte gekennzeichnet, wie sie den oben gemachten Angaben entnommen werden können.

Unter der Akzessibilität eines Aminosäurerests ist erfindungsgemäß zu verstehen, wie gut er in natürlicher Konformation des Moleküls dem umgebenden Lösungsmittel (zumeist Wasser) zugänglich ist. Zur Ermittlung dieser physikalisch-chemischen Eigenschaft wird eine Skala von 0 bis 100% zugrundegelegt, wobei ein Wert von 0% Akzessibilität bedeutet, daß der betreffende Rest dem Solvens nicht zugänglich ist, und 100% für die in einem hypothetischen Pentapeptid GGXGG mögliche Zugänglichkeit steht.

Erfindungsgemäß spiegelt sich diese Moleküleigenschaft darin wieder, daß ein exponierter, sich aus der Oberfläche abhebender Aminosäurerest leichter mit anderen Molekülen in Berührung kommt als einer, der dem Lösungsmittel weniger zugänglich ist. Somit stellen solche Reste bevorzugte Angriffspunkte für die Hydrolyse dar, beispielsweise durch Nukleophile wie Hydroxylionen. Folglich sind diese Reste auch bevorzugte Ansatzpunkte zum Schutz des Moleküls über die vorliegende Erfindung.

Über den bereits genannten SwissPDB-Viewer besteht die Möglichkeit, auch diese Werte für jede Oberflächen-Aminosäure eines globulären Proteins zu berechnen. Die Berechnung der Akzessibilität der Aminosäuren geschieht dort über den Menüpunkt "Select --> Accessible aa", gefolgt von der Eingabe der Zugänglichkeit in Prozent. Danach sind die entsprechenden Aminosäuren ausgewählt und können durch Drücken der "Return"-Taste angezeigt werden. Es ist dabei zusätzlich möglich, die Berechnung über ein selbst erstellbares Programm zu automatisieren, was umso empfehlenswerter ist, je mehr Aminosäuren in die Berechnung einbezogen werden sollen.

Wie in Beispiel 2 dargestellt ist, wurden für die α-Amylase von *Bacillus sp.* A 7-7 (DSM 12368) folgende 41 beziehungsweise 14 N- und Q-Reste ermittelt, deren auf diese Weise bestimmbare Akzessibilität mindestens 10% beträgt, welche jeweils in % in Klammern hinter den genannten Positionen angegeben ist:
N6 (12), N19 (28), N22 (28), N25 (16), N33 (28), N54 (35), N70 (38), N83 (44), N95 (19), N123 (19), N126 (18), N128 (31), N150 (39), N154 (41), N175 (20), N195 (24), N215 (27), N219 (32), N226 (40), N255 (42), N260 (49), N270 (38), N277 (24), N283 (40), N285 (27), N296 (15), N299 (23), N306 (51), N314 (35), N402 (16), N409 (11), N418 (25), N423 (40), N437 (37), N445 (41), N457 (29), N465 (22), N471 (20), N475 (25), N484 (12), N485 (38);
beziehungsweise:
Q53 (12), Q84 (24), Q86 (18), Q98 (29), Q129 (31), Q169 (26), Q172 (53), Q174 (44), Q311 (35), Q319 (38), Q361 (36), Q394 (20), Q401 (14), Q449 (31).

Hierunter besitzen also folgende 32 beziehungsweise 11 eine Akzessibilität von mindestens 20%:
N19 (28), N22 (28), N33 (28), N54 (35), N70 (38), N83 (44), N128 (31), N150 (39), N154 (41), N175 (20), N195 (24), N215 (27), N219 (32), N226 (40), N255 (42), N260 (49), N270 (38), N277 (24), N283 (40), N285 (27), N299 (23), N306 (51), N314 (35), N418 (25), N423 (40), N437 (37), N445 (41), N457 (29), N465 (22), N471 (20), N475 (25), N485 (38);
beziehungsweise:
Q84 (24), Q98 (29), Q129 (31), Q169 (26), Q172 (53), Q174 (44), Q311 (35), 0319 (38), Q361 (36), Q394 (20), Q449 (31).

Hierunter besitzen also folgende 18 beziehungsweise 7 eine Akzessibilität von mindestens 30%:
N54 (35), N70 (38), N83 (44), N128 (31), N150 (39), N154 (41), N219 (32), N226 (40), N255 (42), N260 (49), N270 (38), N283 (40), N306 (51), N314 (35), N423 (40), N437 (37), N445 (41), N485 (38);
beziehungsweise:
Q129 (31), Q172 (53), Q174 (44), Q311 (35), Q319 (38), Q361 (36), Q449 (31).

Diese Gruppierung dient lediglich der Veranschaulichung. Erfindungsgemäß sind die betreffenden Reste mit zunehmender Akzessibilität zunehmend bevorzugt, weil sie zunehmend dem Lösungsmittel ausgesetzt sind und damit um so leichter hydrolysiert werden können.

In einer weiter bevorzugten Ausführungsform handelt es sich bei erfindungsgemäßen α-Amylase-Varianten um solche α-Amylase-Varianten, wobei mehrere der genannten Aminosäureaustausche durchgeführt worden sind, vorzugsweise 2 bis 10, besonders bevorzugt 3 bis 8, ganz besonders bevorzugt 4 bis 6.

Erfindungsgemäß wird angenommen, daß jeder einzelne erfindungsgemäße Austausch für sich genommen einen vorteilhaften Effekt hervorruft. Dennoch kann nicht grundsätzlich von einer Additivität dieser vorteilhaften Effekte aller erfindungsgemäßer Aminosäuresubstitutionen ausgegangen werden. Es ist also im Einzelfall empfehlenswert, eine erfindungsgemäße Mutation nach der anderen vorzunehmen und zu überprüfen, ob damit jeweils eine Stabilitätsverbesserung erzielt worden ist. Da jeweils eine gewisse (wenn auch nicht notwendigerweise additive oder sogar synergistische) Verbesserung zumindest prinzipiell zu erwarten ist, wird vorzugsweise mehr als eine erfindungsgemäße Substitution durchgeführt.

Diesem Vorgehen ist der Natur der Enzyme entsprechend auch eine individuelle Obergrenze gesetzt, die gegebenenfalls experimentell ermiltelt werden muß. Die hierfür mögliche Obergrenze umfaßt lediglich prinzipiell alle auf der Molekoloberfläche gelegenen N- und Q-Reste. Die an dieser Stelle festgesetzte bevorzugte Obergrenze von zehn Austauschen ist zum einen technisch noch gut realisierbar. Zum anderen gehen mit jeder Mutation, auch den erfindungsgemäßen, Änderungen der Moleküleigenschaften einher. Ein Beispiel hierfür sind die oben angegebenen Polaritätswerte für die besonders bevorzugten Austausche gegen S oder T. Es ist anzunehmen, daß für das gesamte Molekül ein allzu drastisches Absenken aller Polaritäten wiederum mit einer Zunahme der Instabilität verbunden ist. Auch dies rechtfertigt die Vorgabe, das jeweilige Optimum experimentell zu ermitteln.

Für die α-Amylase aus *Bacillus sp.* A 7-7 (DSM 12368) haben sich die angegebenen Grenzwerte als in mehrerer Hinsicht vorteilhaft herausgestellt, was einerseits die Wirksamkeit und andererseits die Realisierbarkeit angeht.

Es handelt es sich bei dem Ausgangsmolekül bevorzugt um die α-Amylase aus *Bacillus sp.* A 7-7 (DSM 12368).

So wird die hier beschriebene Erfindung in den Beispielen der vorliegenden Anmeldung insbesondere anhand der α-Amylase aus *Bacillus sp.* A 7-7 (DSM 12368) dargestellt, deren aller Wahrscheinlichkeit nach korrekte DNA- und Aminosäuresequenzen im Sequenzprotokoll der vorliegenden Anmeldung gezeigt sind. Dieses Wildtypenzym besitzt gegenüber anderen etablierten α-Amylasen die in der Anmeldung WO 02/10356 A2 dargestellten Vorteile hinsichtlich eines Einsatzes in Wasch- und Reinigungsmitteln.

In einer weiter bevorzugten Ausführungsform sind erfindungsgemaße α-Amylase-Varianten solche α-Amylase-Varianten, wobei es sich bei dem Ausgangsmolekül um eine α-Amylase handelt, deren Aminosäuresequenz zu der in SEQ ID NO. 2 angegebenen Aminosäuresequenz in den Positionen +1 bis 484 mindestens zu 96% identisch ist, vorzugsweise zu 98%, besonders bevorzugt zu 100%.

Diese α-Amylase aus *Bacillus sp.* A7-7 (DSM 12368) wird In der bereits mehrmals erwähnten internationalen Patentanmeldung WO 02/10356 A2 ausführlich beschrieben. Hierzu sei angemerkt, daß die Nukleotidsequenz und die davon abgeleitete Aminosäuresequenz der aus diesem Stamm erhältlichen α-Amylase aller Wahrscheinlichkeit nach die in SEQ ID NO. 1 beziehungsweise 2 der vorliegenden Anmeldung angegebenen Sequenzen aufweisen. Dies ist eine Korrektur gegenüber den in der Anmeldung WO 02/10356 A2 angegebenen Sequenzen. Auch die in jener Anmeldung beschriebenen Versuche sind mit der aus diesem Stamm erhältlichen, nativen α-Amylase durchgeführt worden.

Die Nukleotidsequenz gemäß SEQ ID NO. 1 versetzt den Fachmann unmittelbar in die Lage, erfindungsgemäße Mutationen vorzunehmen. Wer anstatt auf den hinterlegten Mikroorganismus zurückzugreifen, eine α-Amylase-codierende DNA nach dem Sequenzprotokoll von WO 02/10356 A2 hergestellt hat, kann diese mithilfe einfacher Punktmutageneseverfahren, wie sie auch in den Beispielen der vorliegenden Anmeldung genannt werden, in eine Nukleotidsequenz gemäß SEQ ID NO. 1 der vorliegenden Anmeldung umwandeln.

Die Kultivierungsmöglichkeiten des zugehörigen Mikroorganismus, die Aufreinigung und die enzymatische Charakterisierung der Wildtyp-α-Amylase sind aus jener Anmeldung ebenfalls bekannt und werden in Beispiel 1 der vorliegenden Anmeldung noch einmal zusammengefaßt. Erfindungsgemäße Varianten dieses Enzyms lassen sich in prinzipiell gleicher Weise gewinnen und aufreinigen, wobei - beispielsweise bei der Betrachtung des isoelektrischen Punkts (IEP) - die erfindungsgemäß eingeführten Unterschiede zu berücksichtigen sind.

Weiterhin bevorzugt sind solche erfindungsgemäßen α-Amylase-Varianten, wobei es sich bei dem Ausgangsmolekül um eine α-Amylase-Varlante mit zunehmend bevorzugt 1, 2, 3, 4, 5, 6, 7, 8, 9, 10 oder 11 zusätzlichen Punktmutationen handelt.

Die in SEQ ID NO. 1 und 2 angegebenen Sequenzen unterscheiden sich, wie in Beispiel 1 erläutert wird, in jeweils zwei Positionen von den Angaben in WO 02/10356 A2. Sie stellen nach dem heutigen Kenntnisstand die besten Beschreibungen der α-Amylase aus *Bacillus sp.* A 7-7 (DSM 12368) dar und sind somit besonders bevorzugte Ausgangspunkte zum Einführen erfindungsgemäßer Aminosäureaustausche.

Weiterhin bevorzugte Ausführungsformen der vorliegenden Erfindung sind erfindungsgemäße α-Amylase-Varianten mit den zusätzlichen Punktmutationen in einer oder mehreren der folgenden Positionen: 5, 167, 170, 177, 202, 204, 271, 330, 377, 385 und 445, in der Zählung des maturen Proteins gemäß SEQ ID NO. 2.

So geht aus der nicht vorveröffentlichten deutschen Patentanmeldung DE 10309803.8 hervor, daß in den Positionen -19, 5, 167, 170, 177, 204, 271, 330, 377, 385 und/oder 445 (beziehungsweise 13, 32, 194, 197, 203, 230, 297, 356, 406, 414 und 474 gemäß der Zählung der nicht prozessierten α-Amylase aus *B. amyloliquefaciens*) Punktmutationen zur Verbesserung der Alkaliaktivität von α-Amylasen vorgenommen werden können.

Gegenüber Oxidation stabilisierte α-Amylase-Varianten gehen wie einleitend bereits dargestellt aus der Anmeldung WO 94/18314 A1 hervor, hierunter vor allem in Position 197 (in der Zählung der α-Amylase von *B. licheniformis*), die der Position 202 der α-Amylase gemäß SEQ ID NO. 2 entspricht. Erfindungsgemäße Varianten können durch die darin genannten Austausche, vor allem in Position 202 zusätzlich gegenüber Oxidation stabilisiert werden. Dieser Austausch ist auch deshalb besonders interessant, weil es sich bei dem M202 in der α-Amylase aus *Bacillus sp.* A 7-7 (DSM 12368) um den einzigen Methionin-Rest handelt, der mit einem Akzessibilitätswert von 30% dem Lösungsmittel zu mehr als 10% zugänglich ist (vergleiche Beispiel 2). Hierdurch erklärt sich dessen besondere Oxidationssensitivität sowie der Zusammenhang zur vorliegenden Erfindung.

Bevorzugte Ausführungsformen hiervon stellen solche α-Amylase-Varianten dar, wobei es sich in den Ausgangsenzymen um folgende Punktmutationen handelt: 5A, 167R, 170P, 177L, 202L, 204V, 271D, 330D, 377R, 386S und/oder 445Q.

So sind die Sequenzvariationen -19P, 5A, 167R, 170P, 177L, 204V, 271D, 330D, 377R, 385S und 445Q (beziehungsweise in der Zählung der unprozessierten α-Amylase aus *B. licheniformis* die Austausche zu 13P, 32A, 194R, 197P, 203L, 230V, 297D, 356D. 406R, 414S und 474Q) in der erwähnten Anmeldung DE 10309803.8 als bevorzugte Austausche beschrieben.

In WO 94/18314 A1 wird besonders die oxidationsstabilisierende Wirkung des Austauschs M197L dargestellt, der der Aminosäuresubstitution M202L gemäß SEQ ID NO. 2 entspricht. Erfindungsgemäße Varianten können somit insbesondere durch diese Punktmutation zusätzlich gegenüber Oxidation stabilisiert werden.

Eine weitere Lösung der gestellten Aufgabe und somit einen weiteren Gegenstand der vorliegenden Erfindung bilden Verfahren zur Erhöhung der Stabilität einer α-Amylase gegenüber einer über das Lösungsmittel ausgeübten Hydrolyse gemäß Anspruch 9.

Denn wie erläutert ist erfindungsgemäß ein beträchtlicher Anteil des Aktivitätsverlusts von α-Amylase-haltigen Lösungen auf die Hydrolyse der empfindlichen Oberflächenaminosäuren Asparagin (N) und Glutamin (Q) zurückzuführen. Dies wird als ein Stabilitätsaspekt verstanden. Jedes Verfahren, das der Hydrolyse dieser Aminosäurereste entgegenwirkt, ist somit eines, das die Stabilität der betreffenden Enzympräparation hinsichtlich ihrer Gesamtaktivität erhöht.

Der erfindungsgemäße Ansatz zum Lösen dieses Problems besteht, wie ebenfalls bereits erläutert, darin, eine andere Aminosäure an deren Stelle einzuführen, wofür prinzipiell jede andere andere Aminosäure als Q beziehungsweise N, D oder E geeignet ist.

Verfahren zur Herstellung von Enzymvarianten sind einem Biotechnologen an sich bekannt. Er greift dabei insbesondere auf die für die betreffenden Ausgangsenzyme codierenden Nukleinsäuren zurück, die entsprechend der einzuführenden Aminosäure in dem zugehörigen Codon mutiert wird.

Das Prinzip hierzu wird auch aus Beispiel 3 deutlich: Beispielsweise mit Hilfe des QuikChange-Kit (Fa. Stratagene, Kat.-Nr. 200518) werden nach dem zugehörigen Protokoll Primer synthetisiert, die den zu mutierenden Bereich überdecken und die einzuführende Mutation enthalten (Mismatch-Primer). Die Primersequenzen werden anhand der zugehörigen Nukleotidsequenz unter Berücksichtigung des universellen genetischen Codes entworfen. Nach diesem Prinzip wird geeigneterweise ein die α-Amylase-Sequenz enthaltender Expressionsvektor entsprechend mutagenisiert und nach allgemein bekannten Verfahren in einen zur Expression der Amylase geeigenten Expressionsstamm transformiert. Da die Variante im Vergleich zum Ausgangsmolekül in der Regel nur einige Austausche aufweist, kann man sich bei der Wahl des Expressionsystems, der Kultivierungs- und der Aufarbeitungsverfahren an den für das Ausggangsmolekül etablierten Systemen orientieren. Dabei ist zu berücksichtigen, daß erfindungsgemäß die biophysikalischen Eigenschaften der α-Amylasen verändert werden. Eine dementsprechende Änderung des IEP kann beispielsweise mithilfe einer isolelektrischen Fokussierung überprüft werden.

Bevorzugt sind erfindungsgemäße Verfahren zur Erhöhung der Stabilität einer α-Amylase gegenüber einer über das Lösungsmittel bei erhöhten Temperaturen oder hohen pH-Werten ausgeübten Hydrolyse.

Denn wie erläutert kann die beschriebene Instabilität insbesondere unter diesen Bedingungen beobachtet werden. Dies kann - ohne daß diese These als verbindlich anzusehen ist - damit erklärt werden, daß chemische Reaktionen grundsätzlich bei höheren Temperaturen schneller ablaufen und Biopolymere wie Proteine bei höheren Temperaturen leichter denaturiert werden. Zum anderen ist das bei alkalischen pH-Werten verstärkt auftretende Hydroxylion ein sehr reaktives Nukleophil, insbesondere gegenüber der Carboxylgruppe ebendieser Säureamide.

Ansonsten gelten die oben zu den jeweiligen Enzymen gemachten Aussagen entsprechend. Dies gilt entsprechend auch für die bevorzugten Ausführungsformen; das heißt für diejenigen erfindungsgemäßen Verfahren, mit denen die oben bereits als erfindungsgemäß dargestellten Varianten erhalten werden.

Entsprechend dem oben Gesagten handelt es sich bei bevorzugten erfindungsgemäßen Verfahren um solche, wobei das N durch ein A, G, K, R, S oder T, vorzugsweise durch ein S oder T beziehungsweise das Q durch ein A, G, I, K, R, S oder T, vorzugsweise durch ein S oder T ersetzt wird.

Entsprechend dem oben Gesagten handelt es sich bei weiter bevorzugten erfindungsgemäßen Verfahren um solche, wobei der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% (nicht dem Solvens zugänglich) bis 100% (in einem hypothetischen Pentapeptid GGXGG enthalten) berechnet wird.

Entsprechend dem oben Gesagten handelt es sich bei weiter bevorzugten erfindungsgemäßen Verfahren um solche, wobei wobei mehrere der genannten Aminosäureaustausche durchgeführt werden, vorzugsweise 2 bis 10, besonders bevorzugt 3 bis 8, ganz besonders bevorzugt 4 bis 6.

Hierbei ist die Reihenfolge der einzelnen Substitutionen prinzipiell unerheblich, weil es auf die Eigenschaften des letztlich erhaltenen Moleküls ankommt.

Entsprechend dem oben Gesagten handelt es sich bei weiter bevorzugten erfindungsgemäßen Verfahren um solche, wobei es sich bei dem Ausgangsmolekül um eine α-Amylase handelt, deren Aminosäuresequenz zu der in SEQ ID NO. 2 angegebenen Aminosäuresequenz in den Positionen +1 bis 485 mindestens zu 96% identisch ist, vorzugsweise zu 98%, besonders bevorzugt zu 100%.

Hierunter sind entsprechend dem oben Gesagten Verfahren bevorzugt, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

Entsprechend dem oben Gesagten handelt es sich bei weiter bevorzugten erfindungsgemäßen Verfahren um solche, wobei die eingebrachte α-Amylase eine α-Amylase-Variante mit den zusätzlichen Punktmutationen in einer oder mehreren der folgenden Positionen ist: 5, 167, 170, 177, 202, 204, 271, 330, 377, 385 und 445, in der Zählung des maturen Proteins gemäß SEQ ID NO. 2.

Hierunter sind entsprechend dem oben Gesagten Verfahren bevorzugt, wobei es sich um folgende Punktmutationen handelt: 5A, 167R, 170P, 177L, 202L, 204V, 271D, 330D, 377R, 385S und/oder 445Q.

Einen weiteren Gegenstand der vorliegenden Erfindung bilden Nukleinsäuren, die für eine der oben beschriebenen erfindungsgemäßen α-Amylase-Varianten codieren.

Hierunter sind sowohl RNA- als auch DNA-Moleküle sowie DNA-Analoga zu verstehen, sowohl der codierende als auch der codogene Strang, und zwar in jedem Leseraster. Denn beispielsweise ist es möglich, die mit der Erfindung verbundene Lehre auszunutzen, um über eine interferierende entsprechende RNA eine Regulation entsprechender α-Amylasen vorzunehmen oder die Lebensdauer der genetischen Information zu erhöhen, indem sie in ein *in vivo* langsamer abbaubares DNA-Analogon überführt wird. Die Nukleinsäuren eröffnen, wie an den weiter unten ausgeführten Erfindungsgegenständen zu erkennen ist, der vorliegenden Erfindung gewissermaßen die molekularbiologische Dimension. Sie sind ensprechend dem oben Gesagten entsprechend bevorzugt.

Vorzugsweise sind hierunter DNA-Moleküle zu verstehen. Denn über diese ist es möglich, die erfindungsgemäßen α-Amylase-Varianten über an sich bekannte molekularbiologische Verfahren herzustellen und/oder gegebenenfalls weiterzumodifizieren. Für die oben genannten, im Stand der Technik etablierten α-Amylasen sind die Nukleotidsequenzen in allgemein bekannten Datenbanken (zum Beispiel Genbank oder Swissprot; siehe oben) hinterlegt oder gehen aus den genannten Publikationen hervor. Diese Sequenzen stellen entsprechend bevorzugte Ausgangspunkte für das Einführen erfindungsgemäßer Punktmutationen dar.

Ausgangspunkte für diese Nukleinsäure können auch die im Sequenzprotokoll angegebenen Sequenzinformationen sein, insbesondere für Derivate der α-Amylase aus *Bacillus sp.* A 7-7 (DSM 12368). Eine weitere Alternative besteht darin, eine Gesamt-DNA-Präparation von bestimmten dafür ins Auge gefaßten Mikroorganismen vorzunehmen und über PCR die endogenen α-Amylasegene zu isolieren. Als Primer können prinzipiell die aus SEQ ID NO. 1 ebenfalls abzulesenden 5'- und 3'-Sequenzabschnitte eingesetzt werden.

Die reinen proteincodierenden Abschnitte können beispielsweise auch über eine PCR mutagenisiert, das heißt erfindungsgemäß in ihrer Sequenz verändert werden. Hierfür werden eine PCR mit einer entsprechenden statistischen Fehlerrate durchgeführt, das PCR-Produkt kloniert und die eingeführten Mutationen durch anschließende Sequenzierung verifiziert.

Bevorzugt ist hierunter eine Nukleinsäure, welche sich von einer Nukleinsäure gemäß SEQ ID NO. 1 ableitet, oder von einer Variante hiervon mit zunehmend bevorzugt 1, 2, 3, 4, 5, 6, 7, 8, 9, 10 oder 11 zusätzlichen Punktmutationen zu solchen Mutationen, die zu einem der oben als erfindungsgemäß definierten Aminosäureaustausche führen, das heißt zusätzlich zu solchen Mutationen, durch die mindestens ein auf der Oberfläche des Moleküls gelegener Asparagin- (N-) oder Glutamin-(Q-)rest zu A, C, F, G, H, I, K, L, M, P, R, S, T, V, W oder Y ausgetauscht worden ist, vorzugsweise zusätzlich zu solchen, wobei das N durch ein A, G, K, R, S oder T, vorzugsweise durch ein S oder. T ersetzt worden ist, beziehungsweise zusätzlich zu solchen, wobei das Q durch ein A, G, I, K, R, S oder T, vorzugsweise durch ein S oder T ersetzt worden ist.

Denn wie oben erläutert können in α-Amylase-Moleküle verschiedene Punktmutationen eingeführt werden, die prinzipiell unabhängig voneinander vorteilhafte Wirkungen ausüben können. So können erfindungsgemäße Austausche an solchen Molekülen vorgenommen werden, die insbesondere hinsichtlich spezieller Leistungsaspekte, der Stabilität oder etwa der Allergenizität verbessert worden sind. Dazu gehören insbesondere auch nicht erfindungsgemäße Punktmutationen. Dieser Ansatz zur Erzeugung letztlich erfindungsgemäßer Aminosäureaustausche baut auf den entsprechenden Nukleinsäuren auf, wobei es technisch grundsätzlich irrelevant ist, in welcher Reihenfolge diese verschiedenen Mutationen vorgenommen werden.

Einen weiteren Gegenstand der vorliegenden Erfindung bilden Vektoren, die eine zuvor bezeichnete erfindungsgemäße Nukleinsäure enthalten.

Denn um mit den erfindungsrelevanten Nukleinsäuren umzugehen, und damit insbesondere die erfindungsgemäßen Verfahren durchzuführen und Produktion erfindungsgemäßer Proteine vorzubereiten, werden sie geeigneterweise in Vektoren ligiert. Solche Vektoren sowie die zugehörigen Arbeitsmethoden sind im Stand der Technik ausführlich beschrieben. Vektoren sind in großer Zahl und Variationsbreite, sowohl für die Klonierung als auch für die Expression kommerziell erhältlich. Dazu gehören beispielsweise Vektoren, die sich von bakteriellen Plasmiden, von Bakteriophagen oder von Viren ableiten, oder überwiegend synthetische Vektoren. Ferner werden sie nach der Art der Zelltypen, in denen sie sich zu etablieren vermögen, beispielsweise nach Vektoren für gramnegative, für grampositive Bakterien, für Hefen oder für höhere Eukaryonten unterschieden. Sie bilden geeignete Ausgangspunkte beispielsweise für molekularbiologische und biochemische Untersuchungen, für die Mutagenese der enthaltenen Nukleinsäureabschnitte sowie für die Expression des betreffenden Gens oder zugehörigen Proteins. Insbesondere zur Herstellung von Konstrukten zur Deletion oder Verstärkung der Expression sind sie - wie aus dem hierfür einschlägigen Stand der Technik hervorgeht - praktisch unerläßlich.

Vektoren sind besonders geeignet, um erfindungsgemäße Sequenzvariationen vorzunehmen, beispielsweise über ortsgerichtete Mutagenese, wie dies in Beispiel 3 dargestellt ist.

In einer Ausführungsform handelt es sich bei erfindungsgemäßen Vektoren um Klonierungsvektoren.

Denn Klonierungsvektoren eignen sich neben der Lagerung, der biologischen Amplifizierung oder der Selektion des interessierenden Gens für dessen molekularbiologische Charakterisierung. Gleichzeitig stellen sie transportierbare und lagerfähige Formen der beanspruchten Nukleinsäuren dar und sind auch Ausgangspunkte für molekularbiologische Techniken, die nicht an Zellen gebunden sind, wie beispielsweise die PCR oder *In-vitro*-Mutagenese-Verfahren.

Beispielsweise kann ein Klonierungsvektor, welcher das Gen für eine im Stand der Technik beschriebene α-Amylase trägt, durch Durchführung einer erfindungsgemäßen Mutation in einen erfindungsgemäßen Klonierungsvektor umgewandelt werden.

Vorzugsweise handelt es sich bei erfindungsgemäßen Vektoren um Expressionsvektoren.

Denn derartige Expressionsvektoren sind die Basis dafür, die entsprechenden Nukleinsäuren in biologischen Produktionssystemen zu realisieren und damit die zugehörigen Proteine zu produzieren, da sie *in vivo* die Transkription und Translation, das heißt die Synthese des betreffenden Genprodukts ermöglichen. Bevorzugte Ausführungsformen dieses Erfindungsgegenstands sind Expressionsvektoren, die die zur Expression notwendigen genetischen Elemente tragen, beispielsweise den natürlichen, ursprünglich vor diesem Gen lokalisierten Promotor oder einen Promotor aus einem anderen Organismus. Diese Elemente können beispielsweise in Form einer sogenannten Expressionskassette angeordnet sein. Alternativ können einzelne oder alle Regulationselemente auch von der jeweiligen Wirtszelle bereitgestellt werden. Besonders bevorzugt sind die Expressionsvektoren hinsichtlich weiterer Eigenschaften, wie beispielsweise die optimale Kopienzahl, auf das gewählte Expressionssystem, insbesondere die Wirtszelle (siehe unten) abgestimmt.

In der Regel ist die Bereitstellung eines Expressionsvektors die beste Möglichkeit, ein erfindungsgemäßes Protein vorzugsweise in Kombination mit damit zusammenwirkenden erfindnungsgemäßen Proteinen verstärkt zu bilden und somit die betreffende Aktivität zu erhöhen beziehungsweise einer quantitativen Herstellung zugänglich zu machen.

Einen eigenen Erfindungsgegenstand bilden Zellen, die nach gentechnischer Modifizierung eine der zuvor bezeichneten erfindungsgemäßen Nukleinsäuren enthalten.

Denn diese Zellen enthalten die genetische Information zur Synthese eines erfindungsgemäßen Proteins und kommen dann in erfindungsgemäßen Verfahren zum Einsatz, wenn die betreffenden α-Amylasen biotechnologisch gewonnen werden. Hierunter sind insbesondere diejenigen Zellen gemeint, die nach an sich bekannten Verfahren mit den erfindungsgemäßen Nukleinsäuren versehen worden sind, beziehungsweise die sich von solchen Zellen ableiten. Dafür werden geeigneterweise solche Wirtszellen ausgewählt, die sich vergleichsweise einfach kultivieren lassen und/oder hohe Produktausbeuten liefern.

Sie ermöglichen beispielsweise die Amplifizierung der entsprechenden Gene, aber auch deren Mutagenese, wenn ein *In-vivo*-Mutagenese-Verfahren vorgenommen wird, oder Transkription und Translation und letztlich die biotechnologische Produktion der betreffenden Proteine. Diese genetische Information kann entweder extrachromosomal als eigenes genetisches Element, das heißt bei Bakterien in plasmidaler Lokalisation vorliegen oder in ein Chromosom integriert sein. Die Wahl eines geeigneten Systems hängt von Fragestellungen, wie beispielsweise die Art und Dauer der Lagerung des Gens, beziehungsweise des Organismus oder die Art der Mutagenese oder Selektion ab. Derartige Realisierungsmöglichkeiten sind an sich dem Molekularbiologen bekannt.

Daraus erklärt sich auch die bevorzugte Ausführungsform, nach welcher in einer solchen Zelle die genannte Nukleinsäure Teil eines Vektors ist, insbesondere eines zuvor beschriebenen, erfindungsgemäßen Vektors.

Denn damit sind die oben beschriebenen Vorteile bei der Handhabung, Lagerung, Expression etc. der betreffenden Nukleinsäure verbunden.

Bevorzugt ist unter diesen Zellen jeweils eine Wirtszelle, bei der es sich um ein Bakterium handelt, insbesondere eines; das die gebildete α-Amylase-Variante ins umgebende Medium sekretiert.

Denn Bakterien zeichnen sich durch kurze Generationszeiten und geringe Ansprüche an die Kultivierungsbedingungen aus. Dadurch können kostengünstige Verfahren etabliert werden. Zudem verfügt man bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf etc. gramnegative oder grampositive Bakterien geeignet sein:

Eine zusätzlich vorteilhafte Ausführungsform nimmt darauf Bezug, daß die meisten industriell wichtigen α-Amylasen ursprünglich als Enzyme gefunden worden sind, die von den betreffenden Mikroorganismen, insbesondere Bakterien ins umgebende Medium sekretiert werden. Denn *in vivo* handelt es sich zumeist um Verdauungsenzyme. Dementsprechend ist es vorteilhaft, wenn die industriell hergestellten erfindungsgemäßen Enzyme ebenfalls ins umgebende Medium sekretiert werden. Denn aus diesem können sie ohne Zellaufschluß und damit vergleichsweise leicht aufgearbeitet werden. Dies kann beispielsweise dadurch erreicht werden, daß die betreffenden Gene um entsprechende Sequenzen ergänzt werden - sofern diese nicht ohnehin vorhanden sind - die für ein Leader-Peptid codieren, welches die betreffende Zelle dazu veranlaßt sie auszuschleusen. Eine Alternative bei gramnegativen Bakterien besteht beispielsweise darin, die äußere Membran zur Abgabe von Proteinen partiell zu öffnen, wie dies beispielsweise in der Anmeldung WO 01/81597 A1 beschrieben ist.

In einer bevorzugten Ausführungsform handelt es sich um ein gramnegatives Bakterium, insbesondere eines der Gattungen *Escherichia coli, Klebsiella, Pseudomonas* oder *Xanthomonas,* insbesondere um Stämme von *E. coli* K12, *E. coli* B oder *Klebsiella planticola,* und ganz besonders um Derivate der Stämme *Escherichia coli* BL21 (DE3), *E. coli* RV308, *E. coli* DH5α, E.*coli* JM109, *E. coli* XL-1 oder *Klebsiella planticola* (Rf).

Denn diese Spezies und Stämme sind im Stand der Technik für molekularbiologische Arbeitsschritte und biotechnologische Herstellprozesse etabliert. Sie stehen zudem über allgemein zugängliche Stammsammlungen wie der Deutschen Sammlung von Mikroorganismen und Zellkulturen GmbH, Mascheroder Weg 1b, 38124 Braunschweig (http://www.dsmz.de) oder aus kommerziellen Quellen zur Verfügung. Sie sind darüber hinaus im Zusammenspiel mit ebenfalls in großer Zahl zur Verfügung stehenden übrigen Komponenten wie etwa Vektoren auf spezifische Herstellbedingungen hin optimierbar.

Bei gramnegativen Bakterien, wie beispielsweise *E. coli,* werden eine Vielzahl von Proteinen in den periplasmatischen Raum sekretiert. Dies kann für spezielle Anwendungen vorteilhaft sein. Wie erwähnt sind auch Verfahren bekannt, nach welchen auch gramnegative Bakterien die exprimierten Proteine ausschleusen.

In einer alternativen, nicht minder bevorzugten Ausführungsform handelt es sich um ein grampositives Bakterium, insbesondere eines der Gattungen *Bacillus, Staphylococcus* oder *Corynebacterium,* ganz besonders der Species *Bacillus lentus, B. licheniformis, B. amyloliquefaciens, B. subtilis, B.* globigii oder *B.* alcalophilus, *Staphylococcus carnosus* oder *Corynebacterium glutamicum,* und hierunter wiederum ganz besonders bevorzugt um ein Derivat von *B. licheniformis* DSM 13.

Denn grampositive Bakterien besitzen den gramnegativen gegenüber den grundsätzlichen Unterschied, sekretierte Proteine sogleich in das die Zellen umgebende Nährmedium abzugeben, aus welchem sich, wenn das gewünscht ist, die exprimierten erfindungsgemäßen Proteine direkt aufreinigen lassen. Zudem sind sie mit den meisten Herkunftsorganismen für technisch wichtige Enzyme verwandt oder identisch und bilden meist selbst vergleichbare Enzyme, so daß sie über eine ähnliche Codon-Usage verfügen und ihr Protein-Syntheseapparat naturgemäß entsprechend ausgerichtet ist. Der eine ganz besonders bevorzugte Ausführungsform kennzeichnende *B. licheniformis* DSM 13 ist ebenfalls im Stand der Technik als biotechnologischer Produktionsstamm weit verbreitet.

Eine weitere Ausführungsform der vorliegenden Erfindung bilden Verfahren zur Herstellung einer oben beschriebenen α-Amylase-Variante.

Die im engeren Sinne erfindungsgemäßen Verfahren zur Erhöhung der Stabilität einer α-Amylase gegenüber einer über das Lösungsmittel ausgeübten Hydrolyse sind bereits oben beschrieben worden. Sie bezeichnen die in erster Linie molekularbiologischen Schritte, um derartige Varianten überhaupt ersteinmal zu erzeugen. Die an dieser Stelle definierten, im weiteren Sinne ebenfalls erfindungsgemäßen Verfahren sind diejenigen, die technisch notwendig sind, um die erfindungsgemäßen α-Amylase-Varianten in quantitiven Mengen herzustellen. Es sind also - abgesehen von der eher nur theoretisch relevanten chemischen Synthese der Enzyme - in erster Linie biotechnologische Produktionsverfahren erfindungsgemäßer Varianten gemeint. In- diese werden üblicherweise Mikroorganismenstämme eingebracht, die unter Anwendung an sich bekannter molekularbiologischer Techniken in die Lage versetzt worden sind, α-Amylase-Varianten zu produzieren, die im Laufe der vorliegenden Erfindung als erfindungsgemäß vorteilhaft erkannt worden sind.

Das oben ausgeführte erfindungsgemäße, insbesondere auf einer Mutagenese beruhende Verfahren kann somit als kennzeichnender Verfahrensabschnitt in ein etabliertes biotechnologisches Verfahren zur Herstellung von α-Amylasen eingebracht, das heißt mit einem solchen kombiniert werden.

Als Nachweis für die Bildung eines betreffenden Proteins in einem für die Herstellung eingesetzten Stamm dient ein enzymatischer Nachweis der betreffenden Enzymaktivitäten über an sich bekannte Nachweisreaktionen für α-Amylase-Aktivität. Als Nachweis auf molekularbiologischer Ebene können die im vorliegenden Sequenzprotokoll dargestellten Proteine nach üblichen Methoden synthetisiert und hiergegen Antikörper gebildet werde. Diese Proteine sind dann beispielsweise über entsprechende Western-Blots nachweisbar. Besonders bevorzugt reagieren diese Antikörper auf jene Oberflächenbereiche, die erfindungsgemäß modifiziert worden sind, weil darüber eine Unterscheidbarkeit gegenüber den nichterfindungsgemäßen Varianten gegeben ist.

Das Ziel dieser Verfahren besteht darin, die betreffenden α-Amylasen ihren jeweiligen Anwendungen zuzuführen. Hierfür kann auf alle in der Biotechnologie etablierten Aufarbeitungs-, Reinigungs- und Konfektionierungsschritte zurückgegriffen werden. Dazu gehört beispielsweise das in der Anmeldung WO 2004/067557 A1 beschriebene, auf einer Chromatographie basierende Verfahren zur Veredelung konzentrierter Enzymlösungen, welches sich demzufolge auch erfolgreich auf Amylase-Präparationen anwenden läßt. Hierauf baut die nicht vorveröffentlichte Anmeldung DE 10360841.9 auf, nach welcher die betreffenden, durch unter anderem einen Chromatographie-Schritt erhaltenen Lösungen zu Enzymgranulaten weiterverarbeitet werden können. Weitere ebenfalls vorteilhaft mit der vorliegenden Erfindung zu kombinierende Verfahrensaspekte gehen aus den ebenfalls nicht vorveröffentlichten Anmeldungen DE 102004021384.4 und DE 102004019528 hervor, wonach die Lagerstabilität und Abriebfestigkeit derartiger Granulate durch Einarbeitung von Glycerin oder 1,2-Propandiol beziehungsweise durch eine spezielle Beschichtung erhöht werden können.

Diese Verfahren werden, insbesondere in den Teilschritten, zu denen die Enzympräparation noch in flüssiger Form vorliegt, erfindungsgemäß dadurch verbessert, daß die enthaltene erfindungsgemäße α-Amylase-Variante weniger zur Denaturierung neigt und somit ein geringerer Verlust an Gesamtaktivität eintritt. Dadurch können beispielsweise α-Amylase-Granulate produziert werden, die einen höheren Anteil an Aktivsubstanz enthalten.

Vorzugsweise handelt es sich bei den Herstellverfahren um Verfahren, die unter Einsatz einer oben bezeichneten, erfindungsgemäßen Nukleinsäure erfolgen, vorzugsweise unter Einsatz eines zuvor bezeichneten erfindungsgemäßen Vektors und besonders bevorzugt unter Einsatz einer zuvor bezeichneten erfindungsgemäßen Zelle.

Denn durch die genannten Nukleinsäuren, insbesondere den konkreten im Sequenzprotokoll angegebenen Nukleinsäuren wird die entsprechend bevorzugte genetische Information in mikrobiologisch verwertbarer Form, das heißt für gentechnische Produktionsverfahren zur Verfügung gestellt. Zunehmend bevorzugt ist die Bereitstellung auf einem von der Wirtszelle besonders erfolgreich verwertbaren Vektor beziehungsweise von solchen Zellen selbst. Die betreffenden Produktionsverfahren sind dem Fachmann an sich bekannt.

Ausführungsformen der vorliegenden Erfindung können auf der Grundlage der zugehörigen Nukleinsäuresequenzen auch zellfreie Expressionssysteme sein, bei denen die Proteinbiosynthese *in vitro* nachvollzogen wird. Alle bereits oben ausgeführten Elemente können auch zu neuen Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar, so daß optimale Verfahren für jeden konkreten Einzelfall experimentell ermittelt werden müssen.

Weiterhin bevorzugt sind solche derartigen Verfahren, bei denen die Nukleotidsequenz in einem, vorzugsweise mehreren und besonders bevorzugt allen Codons an die Codon-Usage des Wirtsstamms angepaßt worden ist. Denn die Übertragung eines der genannten Gene in eine weniger verwandte Spezies kann dann besonders erfolgreich zur Synthese des betreffenden Proteins genutzt werden, wenn sie hinsichtlich des Gebrauchs der Codons entsprechend optimiert ist.

Einen eigenen Erfindungsgegenstand stellen Mittel dar, die eine oben beschriebene erfindungsgemäße α-Amylase-Variante enthalten.

Hiermit ist jedes Mittel gemeint, in dem die betreffende α-Amylase in irgendeiner Form zur Anwendung, das heißt in den erwünschten hydrolytischen Kontakt zu ihrem Substrat Stärke oder Stärke-ähnliches Polysaccharid gebracht oder hierfür vorbereitet wird. Der gewünschte Kontakt erfolgt in der Regel in einem wäßrigen Milieu, das günstigerweise auf einen entsprechenden pH-Wert gepuffert ist und gegebenenfalls weitere begünstigende Faktoren enthält. Hierzu gehören beispielsweise weitere Enzyme, die die unmittelbaren Reaktionsprodukte weiter umsetzen, etwa in Hinblick auf Stärkeverflüssigung für die Nahrungsmittel- oder Tierfutterherstellung oder für eine Ethanolproduktion. Ferner gehören hierzu niedermolekulare Verbindungen, die beispielsweise in entstehende Oligosaccharide wie Cyclodextrine eingeschlossen werden, oder niedermolekulare Verbindungen, die die Spaltungsprodukte weiter solubilisieren oder eine den Gesamtprozeß begünstigende Wirkung aufweisen, wie etwa Tenside im Rahmen einer Waschmittelrezeptur. Es kann sich auch um Mittel handeln, in denen die erwünschte amylolytische Aktivität erst mit einer großen Zeitverzögerung induziert werden soll, wie beispielsweise bei temporären Klebeverfahren, nach welchen die α-Amylase-Variante dem betreffenden Klebstoff schon frühzeitig zugesetzt aber erst nach langer Zeit durch Erhöhung des Wassergehalts tatsächlich aktiv wird. Dies trifft beispielsweise auch auf Wasch- und Reinigungsmittel zu, die erst nach einer Phase der Lagerung im Augenblick der Verdünnung in der Waschflotte gegenüber dem Substrat aktiv werden sollen.

Eine Ausführungsform dieses Erfindungsgegenstands sind derartige Mittel, wobei es sich um ein Wasch- oder Reinigungsmittel handelt.

Die erfindungsgemäßen Eigenschaften und wichtigen Inhaltsstoffe für derartige Wasch- und Reinigungsmittel werden weiter unten ausführlich dargestellt. An dieser Stelle erfolgt zunächst eine Übersicht über die wichtigsten und deshalb erfindungsgemäß besonders bevorzugten Ausführungsformen derartiger Wasch- und Reinigungsmittel:
- Ein erfindungsgemäßes Wasch- oder Reinigungsmittel, enthaltend 0,000001 Gewichts-Prozent bis 5 Gew.-%, insbesondere 0,00001 bis 3 Gew.-% der α-Amylase-Variante;
- ein erfindungsgemäßes Wasch- oder Reinigungsmittel, welches zusätzlich andere Enzyme enthält, insbesondere hydrolytische Enzyme oder Oxidoreduktasen, besonders bevorzugt weitere Amylasen, Proteasen, Lipasen, Cutinasen, Hemicellulasen, Cellulasen, β-Glucanasen, Oxidasen, Peroxidasen, Perhydrolasen und/oder Laccasen;
- ein erfindungsgemäßes Wasch- oder Reinigungsmittel, wobei die α-Amylase-Variante durch einen der sonstigen Bestandteile des Mittels stabilisiert und/oder in ihrem Beitrag zur Wasch-, beziehungsweise Reinigungsleistung des Mittels gesteigert wird;
- ein erfindungsgemäßes Wasch- oder Reinigungsmittel, welches insgesamt fest ist, vorzugsweise nach einem Kompaktierungsschritt für mindestens eine der enthaltenen Komponenten, besonders bevorzugt, daß es insgesamt kompaktiert ist;
- ein erfindungsgemäßes Wasch- oder Reinigungsmittel, welches insgesamt flüssig, gelförmig oder pastös ist, vorzugsweise unter Verkapselung für mindestens eine der enthaltenen Komponenten; besonders bevorzugt unter Verkapselung mindestens eines der enthaltenen Enzyme, ganz besonders bevorzugt unter Verkapselung der α-Amylase-Variante.

Ein in einem erfindungsgemäßen Mittel enthaltenes Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Bevorzugte erfindungsgemäße Mittel enthalten zu diesem Zweck Stabilisatoren.

Eine Gruppe von Stabilisatoren sind reversible Proteaseinhibitoren. Häufig werden hierfür Benzamidin-Hydrochlorid, Borax, Borsäuren, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Auch Di-Glycerinphosphat schützt gegen Denaturierung durch physikalische Einflüsse. Ebenso werden Calcium- und/oder Magnesiumsalze eingesetzt, wie beispielsweise Calciumacetat oder Calcium-Formiat.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind lineare C₈-C₁₈ Polyoxyalkylene. Auch Alkylpolyglycoside können die enzymatischen Komponenten des erfindungsgemäßen Mittels stabilisieren und vermögen vorzugsweise, diese zusätzlich in ihrer Leistung zu steigern. Vernetzte N-haltige Verbindungen erfüllen vorzugsweise eine Doppelfunktion als Soil-release-Agentien und als EnzymStabilisatoren. Hydrophobes, nichtionisches Polymer stabilisiert insbesondere eine gegebenenfalls enthaltene Cellulase.

Reduktionsmittel und Antioxidantien erhöhen die Stabilität der Enzyme gegenüber oxidativem Zerfall; hierfür sind beispielsweise schwefelhaltige Reduktionsmittel geläufig. Andere Beispiele sind Natrium-Sulfit und reduzierende Zucker.

Besonders bevorzugt werden Kombinatonen von Stabilisatoren eingesetzt, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird günstigerweise durch die Kombination mit Borsäure und/oder Borsäurederivaten und Polyolen gesteigert und noch weiter durch die zusätzliche Wirkung von zweiwertigen Kationen, wie zum Beispiel Calcium-lonen.

Erfindungsgemäße Mittel sind in einer bevorzugten Ausführungsform dadurch gekennzeichnet, daß sie, beispielsweise um die enthaltenen Wirkstoffe zeitlich oder räumlich voneinander getrennt freizusetzen, aus mehr als einer Phase bestehen. Dabei kann es sich um Phasen in verschiedenen, insbesondere aber um Phasen in denselben Aggregatzuständen handeln.

Erfindungsgemäße Mittel, die aus mehr als einer festen Komponente zusammengesetzt sind, können auf einfache Weise dadurch hergestellt werden, daß verschiedene feste Komponenten, insbesondere Pulver, Granulate oder Extrudate mit verschiedenen Inhaltsstoffen und/oder unterschiedlichem Freisetzungsverhalten in insgesamt loser Form miteinander vermischt werden. Die Herstellung erfindungsgemäßer fester Mittel aus einer oder mehreren Phasen kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 0 486 592 bekanntes, einen Extrusionschritt aufweisendes Verfahren bevorzugt. Eine weitere bevorzugte Herstellung mit Hilfe eines Granulationsverfahrens ist in der europäischen Patentschrift EP 0 642 576 beschrieben.

Proteine können für feste Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Die verkapselte Form bietet sich an, um die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden.

Es können auch mindestens zwei feste Phasen miteinander verbunden vorliegen. So besteht eine Möglichkeit, ein festes erfindungsgemäßes Mittel zur Verfügung zu stellen, in dem Verpressen oder Kompaktieren zu Tabletten. Solche Tabletten können ein- oder mehrphasig sein. Damit bietet auch diese Darreichungsform die Möglichkeit, ein festes erfindungsgemäßes Mittel mit zwei festen Phasen vorzulegen. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN/cm², vorzugsweise bei 60 bis 70 kN/cm² verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN/cm², insbesondere bei 10 bis 15 kN/cm² durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind.

Besonders vorteilhaft ist es, wenn in mehrphasigen Mitteln wenigstens eine der Phasen ein Amylase-sensitives Material, insbesondere Stärke enthält oder von diesem zumindest teilweise umgeben oder beschichtet ist. Auf diese Weise wird diese Phase mechanisch stabilisiert und/oder gegen Einflüsse von außen geschützt und gleichzeitig über eine in der Waschflotte wirksame Amylase angegriffen, so daß die Freisetzung der Inhaltsstoffe erleichtert wird.

Ebenfalls bevorzugte erfindungsgemäße Mittel sind dadurch gekennzeichnet, daß sie insgesamt flüssig, gelförmig oder pastös vorliegen. Die enthaltenen Proteine, vorzugsweise ein erfindungsgemäßes Protein, werden solchen Mitteln bevorzugt ausgehend von einer nach dem Stand der Technik durchgeführten Proteingewinnung und Präparation in konzentrierter wäßriger oder nichtwäßriger Lösung, beispielsweise in flüssiger Form, etwa als Lösung, Suspension oder Emulsion, aber auch in Gelform oder verkapselt oder als getrocknetes Pulver zugesetzt. Derartige erfindungsgemäße Wasch- oder Reinigungsmittel in Form von Lösungen in üblichen Lösungsmitteln werden in der Regel durch einfaches Mischen der Inhaltsstoffe hergestellt, die in Substanz oder als Lösung in einen automatischen Mischer gegeben werden können.

Eine Ausführungsform der vorliegenden Erfindung sind solche flüssigen, gelförmigen oder pastösen Mittel, denen ein erfndungswesentliches Protein und/oder eines der anderen enthaltenen Proteine und/oder einer der anderen enthaltenene Inhaltsstoffe verkapselt, vorzugsweise in Form von Mikrokapseln zugesetzt worden ist. Besonders bevorzugt sind darunter solche mit Kapseln aus amylasesensitivem Material. Solch eine gemeinsame Verwendung von Amylase-sensitiven Materialien und dem erfindungswesentlichen amylolytischen Enzym in einem Wasch- oder Reinigungsmittel kann Synergieeffekte zeigen, etwa dergestalt daß das stärkespaltende Enzym die Spaltung der Mikrokapseln unterstützt und somit den Freisetzungsprozeß der verkapselten Inhaltsstoffe steuert, so daß deren Freisetzung nicht während der Lagerung und/oder nicht zu Beginn des Reinigungsvorgangs, sondern erst zu einem bestimmten Zeitpunkt erfolgt. Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen der vorliegenden Erfindung darstellen.

Ein vergleichbarer Effekt ist dann gegeben, wenn sich die Inhaltsstoffe des Wasch- oder Reinigungsmittels auf mindestens zwei unterschiedliche Phasen verteilen, beispielsweise zwei oder mehr feste, miteinander verbundene Phasen eines tablettenförmigen Wasch- oder Reinigungsmittels, oder verschiedene Granulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert. Der Einsatz des erfindungswesentlichen Enzyms für diesen Zweck stellt somit eine bevorzugte Ausführungsform der vorliegenden Erfindung dar.

Im Rahmen entsprechender Mittel können erfindungsgemäße α-Amylase-Varianten zur Aktivierung der eigenen oder anderer Phasen dienen, wenn sie allein oder zusammen mit mindestens einem anderen reinigungsaktiven oder die Reinigungswirkung unterstützenden Wirkstoff in einem aus mehr als einer Phase bestehenden Wasch- oder Reinigungsmittel bereitgestellt werden. In entsprechender Weise können sie auch dazu dienen, Inhaltsstoffe aus Kapseln freizusetzen, wenn sie oder ein anderer Wirkstoff in einem Wasch- oder Reinigungsmittel in verkapselter form bereitgestellt wird.

Einen weiteren Erfindungsgegenstand stellen Verfahren zur Reinigung von Textilien oder von harten Oberflächen dar, die dadurch gekennzeichnet sind, daß in wenigstens einem der Verfahrensschritte eine oben beschriebene erfindungsgemäße α-Amylase-Variante aktiv wird.

Denn in dieser Ausführungsform wird die Erfindung dadurch realisiert, daß die erfindungsgemäß verbesserten enzymatischen Eigenschaften, insbesondere die erhöhte Stabilität gegen Multimerisierung prinzipiell jedem Reinigungsverfahren zugute kommen, beispielsweise dadurch, daß auch während der Anwendung weniger Enzym durch Aggregation verlorengeht. Jedes Reinigungsverfahren wird um die betreffende Aktivität bereichert, wenn sie in wenigstens einem Verfahrensschritt zugesetzt wird. Derartige Verfahren werden beispielsweise mit Maschinen wie gängigen Haushaltsgeschirrspülmaschinen oder Haushaltswaschmaschinen verwirklicht. Bevorzugte Verfahren werden entsprechend den oben gemachten Angaben entsprechend bevorzugt.

Weiter bevorzugt sind derartige Verfahren, die dadurch gekennzeichnet sind, daß die α-Amylase-Variante über ein oben beschriebenes erfindungsgemäßes Mittel eingesetzt wird.

Besonders bevorzugt ist jedes Verfahren, das dadurch gekennzeichnet ist, daß die α-Amylase in dem betreffenden Verfahrensschritt in einer Menge von 0,01 mg bis 400 mg pro entsprechendem Verfahrensschritt eingesetzt wird, vorzugsweise von 0,02 mg bis 300 mg, besonders bevorzugt von 0,03 mg bis 100 mg.

Günstigenfalls ergeben sich dabei Konzentrationswerte von 0,0005 bis 20 mg pro I, bevorzugt 0,005 bis 10 mg pro I, besonders bevorzugt 0,005 bis 8 mg des amylolytischen Proteins pro I Waschflotte. Die Proteinkonzentration kann mit Hilfe bekannter Methoden, zum Beispiel den oben genannten BCA- oder Biuret-Verfahren bestimmt werden.

Entsprechend den bisherigen Ausführungen wird die vorliegende Erfindung auch durch die Verwendung erfindungsgemäßer α-Amylase-Varianten realisiert. Denn auch hierbei kommen die günstigen Eigenschaften der betreffenden Enzyme zur Wirkung. Diese gelten insbesondere für Reinigungszwecke.

Einen eigenen Erfindungsgegenstand stellt somit die Verwendung einer der oben beschriebenen erfindungsgemäßen α-Amylase-Variante zur Reinigung von Textilien oder von harten Oberflächen dar.

Dabei kann es als einzige wirksame Komponente eingesetzt werden. Vorzugsweise geschieht dies zusammen mit mindestens einem anderen reinigungsaktiven oder die Reinigungswirkung unterstützenden Wirkstoff.

Bevorzugt ist somit eine derartige Verwendung, die dadurch gekennzeichnet ist; daß die α-Amylase-Variante über ein oben beschriebenes erfindungsgemäßes Mittel eingesetzt wird.

Günstigerweise ist eine solche Verwendung dadurch gekennzeichnet, daß pro Anwendung, vorzugsweise pro Anwendung in einer Geschirrspülmaschine oder einer Waschmaschine 0,01 mg bis 400 mg der α-Amylase-Variante eingesetzt werden, bevorzugt 0,02 mg bis 300 mg, besonders bevorzugt 0,03 mg bis 100 mg.

Denn hierdurch ergeben sich in der Waschflotte günstigerweise die oben angegebenen Konzentrationswerte. Diese Dosierung kann, je nach Reinigungsproblem vom Hersteller des Mittels oder vom Endverbraucher vorgenommen werden.

Eine weitere Ausführungsform stellt die Verwendung einer erfindungsgemäßen α-Amylase-Variante zur Behandlung von Rohmaterialien oder Zwischenprodukten in der Textilherstellung dar, insbesondere zum Entschlichten von Baumwolle.

Rohmaterialien und Zwischenprodukte der Textilherstellung, beispielsweise für solche auf Baumwollbasis, werden im Rahmen ihrer Herstellung und Weiterverarbeitung mit Stärke ausgerüstet, um eine bessere Verarbeitung zu ermöglichen. Dieses sowohl auf Garne, auf Zwischenprodukte, als auch auf Textilien angewendete Verfahren nennt man Schichten (sizing). Zur Entfernung der Schlichte, also der stärkehaltigen Schutzschicht (Entschlichten, desizing) sind erfindungsgemäße amylolytische Proteine geeignet, und zwar insbesondere deshalb, weil sie während des Einwirkens in einem flüssigen Medium gegen Multimerisierung stabilisiert sind.

Die nachfolgenden Beispiele erläutern die vorliegende Erfindung weiter.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme, Baukästen (Kits) und Geräte werden nach den Angaben der jeweiligen Hersteller eingesetzt.

### Beispiel 1

### Kultivierung von Bacillus sp. A 7-7 (DSM 12368)

Der Mikroorganismus *Bacillus sp.* A 7-7 ist unter der Hinterlegungsnummer DSM 12368 bei der Deutschen Sammlung von Mikroorganismen und Zellkulturen GmbH, Mascheroder Weg 1b, 38124 Braunschweig (http://www.dsmz.de) hinterlegt. Er wird in der Anmeldung WO 02/10356 A2 beschrieben. Die DNA- und Aminosäuresequenzen der von dieser (hinterlegten) Spezies gebildeten α-Amylase unterscheiden sich in folgenden zwei Positionen von den Sequenzen, die im Sequenzprotokoll von WO 02/10356 A2 dargestellt sind: Die zugehörige DNA verfügt in den Nukleinsäure-Positionen 805-807 gemäß SEQ ID NO. 1 der vorliegenden Anmeldung über das Triplett gat, welches für die Aminosäure D (in Aminosäureposition 236) codiert, und in den Positionen 1156-1158 über das Triplett tat, welches für die Aminosäure Y (in Position 353) codiert. (In SEQ ID NO. 1 beziehungsweise 2 von WO 02/10356 A2 sind an den entsprechenden Stellen die Tripletts ggt für G beziehungsweise tgt für C angegeben.)

Über allgemein bekannte Verfahren zur Punktmutagenese, beispielsweise mit Hilfe des Quickchange^{®}-Kit der Firma Stratagene, La Jolla, USA (siehe unten), und anhand von SEQ ID NO. 1 abgeleiteter Primer, ist es möglich, diese α-Amylase in eine andere zu überführen.

Die Kultivierung von *Bacillus sp.* A 7-7 (DSM 12368) ist in WO 02/10356 A2 beschrieben. Ein geeignetes Medium ist YPSS-Medium, enthaltend 15 g/l lösliche Stärke, 4 g/l Hefeextrakt, 1 g/l K₂HPO₄ und 0,5 g/l MgSO₄ x 7 H₂O, wobei der pH-Wert nach dem Autoklavieren mit 20%iger Natriumcarbonatlösung auf 10,3 eingestellt wird. Hieraus kann, wie ebenfalls in WO 02/10356 A2 dargestellt ist, die betreffende α-Amylase gewonnen werden. Somit sind diese α-Amylase und die davon abgeleiteten Varianten über allgemein bekannte Verfahren zumindest im Labormaßstab herstellbar.

### Beispiel 2

### Homologiemodelling und Auswahl der erfindungsgemäß austauschbaren Aminosäuren

### Homologiemodelling

Das Homologiemodelling wurde für die α-Amylase aus *Bacillus sp.* A 7-7 (DSM 12368) wie in der Beschreibung dargestellt über die RSCB-Protein-Databank (zugänglich über das Max-Delbrück-Zentrum in Berlin, Deutschland) durchgeführt. Dabei wurden bei der Suche mit der Proteinsequenz folgende Strukturen gefunden: die α-Amylase aus *B. licheniformis* (RCSB-PDB-Datenbankeintrag: 1BLI), eine chimäre α-Amylase aus denen von *B. amyloliquefaciens* und *B. licheniformis* in nativer Struktur(1 E3X, 1 E43), ein Acarbose-Komplex derselben chimären α-Amylase (1E3Z), ein Ths/Maltotriose-Komplex derselben chimären α-Amylase (1 E40) und eine kinetisch stabilisierte Variante der α-Amylase aus *B*. *licheniformis* (1OB0).

Durch Übereinanderlagerung (Superimposition) dieser Strukturen im SwissPDB-Viewer wurde eine Leitstruktur erstellt, auf die dann die Proteinsequenz der ALBA aufmodelliert wurde. Anschließend wurde die Orientierung der Seitenketten in der ALBA-Struktur korrigiert und eine Energieminimierung durchgeführt. Die einzelnen Schritte sind dem erwähnten Benutzerhandbuch zu entnehmen und erfolgten gemäß den Standardeinstellungen des Programms.

Insgesamt liegen auf der Oberfläche des 484 Aminosäuren umfassenden Moleküls folgende 407 Aminosäuren (Sie werden als solche über eine Akzessibilität von mindestens 1 definiert; zur Akzessibilität: siehe Beschreibung und nachfolgenden Abschnitt):
T5, N6, G7, T8, M9, Q11, Y12, E14, W15, Y16, L17, P18, N19, D20, G21, N22, H23, W24, N25, R26, R28, S29, D30, A31, S32, N33, K35, D36, K37, G38, 139, T40, A41, W43, P46, A47, W48, K49, G50, A51, S52, Q53, N54, D55, V56, G57, Y58, G59, A60, Y61, D62, L63, Y64, L66, G67, E68, F69, N70, Q71, K72, G73, T74, V75, R76, T77, K78, Y79, G80, T81, R82, N83, Q84, L85, Q86, A87, V89, T90, A91, K93, S94, N95, G96, Q98, V99, Y100, V103, M105, N106, H107, K108, G110, A111, D112, A113, T114, E115, W116, V117, R118, V120, E121, V122, N123, P124, S125, N126, R127, N128, Q129, E130, V131, S132, G133, D134, Y135, T136, I137, E138, W140, K142, F143, D144, F145, P146, G147, R148, G149, N150, T151, K152, S153, N154, F155, K156, W157, R158; W159, Y160, H161, D166, W167, D168, Q169, S170, R171, Q172, L173, Q174, N175, R176, I177, Y178, K179, R181, G182, D183, G184, K185, G186, W187, W189, E190, V191, D192, T193, E194, N195, G196, N197, Y198, D199, Y200, L201, M202, Y203, 1206, D207, M208, D209, H210, P211, E212, V214, N215, E216, L217, R218, N219, V222, W223, T225, N226, T227, L228, G229, L230, D231, F233, R234, 1235, G236, A237, K239, H240, 1241, K242, Y243, S244, F245, T246, R247, D248, W249, L250, T251, H252, V253, R254, N255, T256, T257, G258, K259, N260, M261, F262, A263, E266, F267, W268, K269, N270, D271, I272, G273, A274, I275, E276, N277, S280, K281, N283, W284, N285, H286, S287, V288, F289, P292, L293, Y295, N296, L297, Y298, N299, S301, R302, S303, G304, G305, N306, Y307, D308, M309, R310, Q311, 1312, F313, N314, G315, V318, Q319, R320, H321, P322, T323, H324, T327, F328, V329, D330, N331, H332, D333, Q335, P336, E337, E338, A339, L340, E341, S342, F343, E345, E346, W347, F348, K349, P350, L351, C353, L355, T356, L357, R359, D360, Q361, G362, Y363, S365, V366, F367, Y368, D370, Y371, Y372, G373, 1374, P375, T376, H377, G378, P380, A381, M382, K383, S384, K385, I386, D387, P388, L390, E391, R393, Q394, K395, Y396, Y398, G399, K400, Q401, N402, D403, Y404, L405, D406, H407, H408, N409, M410, R415, E416, G417, N418, T419, A420, H421, P422, N423, S424, M430, D432, G433, P434, G435, G436, N437, K438, W439, Y441, G443, R444, N445, K446, A447, G448, Q449, V450, W451, R452, D453, 1454, T455, G456, N457, R458, S459, G460, T461, V462, T463, 1464, N465, A466, D467, W469, N471, S473, V474, N475, G476, G477, S478, V479, V483, N484, N485

Hierunter befinden sich also folgende 44 Asparaginreste:
N6, N19, N22, N25, N33, N54, N70, N83, N95, N106, N123, N126, N128, N150, N154, N175, N195, N197, N215, N219, N226, N255, N260, N270, N277, N283, N285, N296, N299, N306, N314, N331, N402, N409, N418, N423, N437, N445, N457, N465, N471, N475, N484, N485.

Ferner befinden sich hierunter folgende 17 Glutaminreste:
Q11, Q53, Q71, Q84, Q86, Q98, Q129, Q169, Q172, Q174, Q311, Q319, Q335, Q361, Q394, Q401, Q449.

Diese Asparagin- und Glutaminreste sind in in der Abbildung der α-Amylase von *Bacillus sp.* A 7-7 (DSM 12368) in den Figuren 1 und 2 farblich hervorgehoben.

### Berechnung der Solvenszugänglichkeit (Akzessibilität)

Aufbauend auf diesen Ergebnissen wurde nun die Berechnung der Solvenszugänglichkeit (Akzessibilität) der zuvor beschriebenen auf der Oberfläche befindlichen Aminosäurereste N und Q durchgeführt. Hierfür wurde wiederum der bereits erwähnte SwissPdb-Viewer eingesetzt, unter Einhaltung der Standard-Paramter des Programms. Dadurch wurden für die gefundenen Aminosäurereste folgende Werte für die Solvenszugänglichkeit ermittelt, die jeweils in % in Klammern hinter den genannten Positionen angegeben sind:
N6 (12), N19 (28), N22 (28), N25 (16), N33 (28), N54 (35), N70 (38), N83 (44), N95 (19), N106 (1), N123 (19), N126 (18), N128 (31), N150 (39), N154 (41), N175 (20), N195 (24), N197 (3), N215 (27), N219 (32), N226 (40), N255 (42), N260 (49), N270 (38), N277 (24), N283 (40), N285 (27), N296 (15), N299 (23), N306 (51), N314 (35), N331 (6), N402 (16), N409 (11), N418 (25), N423 (40), N437 (37), N445 (41), N457 (29), N465 (22), N471 (20), N475 (25), N484 (12), N485 (38);
beziehungsweise:
Q11 (4), Q53 (12), Q71 (5), Q84 (24), Q86 (18), Q98 (29), Q129 (31), Q169 (26), Q172 (53), Q174 (44), Q311 (35), Q319 (38), Q335 (3), Q361 (36), Q394 (20), Q401 (14), Q449 (31).

Hierunter besitzen also folgende 41 beziehungsweise 14 eine Akzessibilität von mindestens 10%:
N6 (12), N19 (28), N22 (28), N25 (16), N33 (28), N54 (35), N70 (38), N83 (44), N95 (19), N123 (19), N126 (18), N128 (31), N150 (39), N154 (41), N175 (20), N195 (24), N215 (27), N219 (32), N226 (40), N255 (42), N260 (49), N270 (38), N277 (24), N283 (40), N285 (27), N296 (15), N299 (23), N306 (51), N314 (35), N402 (16), N409 (11), N418 (25), N423 (40), N437 (37), N445 (41), N457 (29), N465 (22), N471 (20), N475 (25), N484 (12), N485 (38);
beziehungsweise:
Q53 (12), Q84 (24), Q86 (18), Q98 (29), Q129 (31), Q169 (26), Q172 (53), Q174 (44), Q311 (35), Q319 (38), Q361 (36), Q394 (20), Q401 (14), Q449 (31).

Hierunter besitzen also folgende 32 beziehungsweise 11 eine Akzessibilität von mindestens 20%:
N19 (28), N22 (28), N33 (28), N54 (35), N70 (38), N83 (44), N128 (31), N150 (39), N154 (41), N175 (20), N195 (24), N215 (27), N219 (32), N226 (40), N255 (42), N260 (49), N270 (38), N277 (24), N283 (40), N285 (27), N299 (23), N306 (51), N314 (35), N418 (25), N423 (40), N437 (37), N445 (41), N457 (29), N465 (22), N471 (20), N475 (25), N485 (38);
beziehungsweise:
Q84 (24), Q98 (29), Q129 (31), Q169 (26), Q172 (53), Q174 (44), Q311 (35), Q319 (38), Q361 (36), Q394 (20), Q449 (31).

Hierunter besitzen also folgende 18 beziehungsweise 7 eine Akzessibilität von mindestens 30%:
N54 (35), N70 (38), N83 (44), N128 (31), N150 (39), N154 (41), N219 (32), N226 (40), N255 (42), N260 (49), N270 (38), N283 (40), N306 (51), N314 (35), N423 (40), N437 (37), N445 (41), N485 (38);
beziehungsweise:
Q129 (31), Q172 (53), Q174 (44), Q311 (35), Q319 (38), Q361 (36), Q449 (31).

### Beispiel 3

### Ortsgerichtete Mutagenese

Die im vorangegangenen Beispiel ermittelten Positionen dienen für die α-Amylase aus *Bacillus sp.* A 7-7 (DSM 12368) als Ausgangspunkt für Punktmutationen über ortsgerichtete Mutagenese, das heißt zum Einführen einer anderen Aminosäure für die betreffende(n) Positione(n). Sie wird beispielsweise mit Hilfe des QuikChange-Kit (Fa. Stratagene, Kat.-Nr. 200518) nach dem zugehörigen Protokoll durchgeführt. Die Primer können anhand der in SEQ ID NO. 1 angegebenen DNA- und Aminosäuresequenzen entworfen werden, wobei das jeweilige Codon entsprechend der einzuführenden Aminosäure geändert wird.

Nach diesem Prinzip wird geeigneterweise ein die α-Amylase-Sequenz enthaltender Expressionsvektor entsprechend mutagenisiert und nach allgemein bekannten Verfahren in einen Expressionsstamm, im vorliegenden Beispiel in *B. subtilis* transformiert.

### Beispiel 4

### Gewinnung und Reinigung der Amylasemutanten

Die Anzucht Amylase-positiver *B. subtilis*-Stämme erfolgt in dem in Beispiel 1 genannten YPSS-Medium. Dieses Vorgehen sowie die Aufreinigung des von diesen Stämmen gebildeten Enzyms erfolgt gemäß der Beschreibung in WO 02/10356 A2. Daraus geht auch die Bestimmung der amylolytisclien Aktivität des gereinigten Enzyms gemäß der sogenannten DNS-Methode hervor. Im folgenden dient die auf diese Weise bestimmbare Aktivität als Parameter für die Stabilität des Enzyms unter jeweils verschiedenen Bedingungen.

### Bestimmung der Solvens-Stabilität

Die Solvens-Stabilität der Varianten im Vergleich zu den jeweiligen Ausgangsenzymen, das heißt den Wildtypenzymen beziehungsweise denen, die gerade nicht die erfindungsgemäßen Austausche aufweisen, wird geeigneterweise bei 30minütiger Inkubation bei 40°C in einem Puffer mit einem pH-Wert von 11 gemessen. Anschließend erfolgt der in der Anmeldung WO 02/10356 A2 beschriebene Aktivitätstest, in dem erfindungsgemäße Varianten signifikant bessere Werte ergeben.

### Beschreibung der Figuren

- Figur 1:: Darstellung der Conolly-Oberfläche der α-Amylase aus *Bacillus sp.* A 7-7 (DSM 12368); Vorderansicht.

Die Darstellung wurde mithilfe des Swiss-Pdb-Viewers erstellt (Swiss Institute of Bioinformatics; http://us.expasy.org/spdbv/; Details: siehe Text).
Farbcodierung:

| | |
|---|---|
| hellgrau: | Asparaginreste |
| dunkelgrau: | Glutaminreste |

- Figur 2:: Darstellung der Conolly-Oberfläche der α-Amylase aus *Bacillus sp.* A 7-7 (DSM 12368); Rückansicht.
Weitere Angaben: siehe Figur 1 und Text.
- Figur 3:: Alignment der α-Amylase aus *Bacillus sp.* A 7-7 (DSM 12368; SEO ID NO. 2) mit den wichtigsten anderen α-Amylasen aus dem Stand der Technik, jeweils über den Bereich des maturen Proteins, das heißt den Positionen 32 bis 516 gemäß SEQ ID NO. 2.

Die individuelle Zählung kann anhand der zu Beginn jeder Zeile hinter dem jeweiligen Namen angegebenen Positionsnummer für die jeweils erste gezeigte Aminosäure nachvollzogen werden.
Darin bedeuten:

| | |
|---|---|
| A 7-7: | α-Amylase aus *Bacillus sp.* A 7-7(DSM 12368; SEQ ID NO. 2) |
| S707: | α-Amylase aus *Bacillus sp.* #707 |
| LAMY: | α-Amylase aus *Bacillus sp.* KSM-AP1378 |
| BAA: | α-Amylase aus *B. amyloliquefaciens* |
| BLA: | α-Amylase aus *B. licheniformis* |
| BStA: | α-Amylase aus *B. stearothermophilus* |
| MK716: | α-Amylase aus *Bacillus sp.* MK716 |
| TS-23: | α-Amylase aus *Bacillus sp.* TS-23 |
| K38: | α-Amylase aus *Bacillus* KSM-K38 |

## Patentansprüche

1. α-Amylase-Variante mit mindestens einem Aminosäureaustausch gegenüber dem Ausgangsmolekül, wodurch mindestens ein auf der Oberfläche des Moleküls gelegener Asparagin- (N-) oder Glutamin-(Q-)rest zu A, C, F, G, H, I, K, L, M, P, R, S, T, V, W oder Y ausgetauscht worden ist, wobei es sich bei dem Ausgangsmolekül um folgende α-Amylase handelt: α-Amylase aus *Bacillus sp.* A 7-7 (DSM 12368).

2. α-Amylase-Variante nach Anspruch 1, wobei das N durch ein A, G, K, R, S oder T, vorzugsweise durch ein S oder T beziehungsweise das Q durch ein A, G, I, K, R, S oder T, vorzugsweise durch ein S oder T ersetzt worden ist.

3. α-Amylase-Variante nach einem der Ansprüche 1 oder 2, in der der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% (nicht dem Solvens zugänglich) bis 100% (in einem hypothetischen Pentapeptid GGXGG enthalten) berechnet wird.

4. α-Amylase-Variante nach einem der Ansprüche 1 bis 3, wobei mehrere der genannten Aminosäureaustausche durchgeführt worden sind, vorzugsweise 2 bis 10, besonders bevorzugt 3 bis 8, ganz besonders bevorzugt 4 bis 6.

5. α-Amylase-Variante nach einem der Ansprüche 1 bis 4, wobei es sich bei dem Ausgangsmolekül um eine α-Amylase handelt, deren Aminosäuresequenz zu der in SEQ ID NO. 2 angegebenen Aminosäuresequenz in den Positionen +1 bis 484 mindestens zu 96% identisch ist, vorzugsweise zu 98%, besonders bevorzugt zu 100%.

6. α-Amylase-Variante nach einem der Ansprüche 1 bis 5, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

7. α-Amylase-Variante nach Anspruch 6 mit den zusätzlichen Punktmutationen in einer oder mehreren der folgenden Positionen: 5, 167, 170, 177, 202, 204, 271, 330, 377, 385 und 445, in der Zählung des maturen Proteins gemäß SEQ ID NO. 2.

8. α-Amylase-Variante nach Anspruch 7, wobei es sich um folgende Punktmutationen handelt: 5A, 167R, 170P, 177L, 202L, 204V, 271 D, 330D, 377R, 385S und/oder 445Q.

9. Verfahren zur Erhöhung der Stabilität einer α-Amylase gegenüber einer über das Lösungsmittel ausgeübten Hydrolyse, wodurch mindestens ein auf der Oberfläche des Moleküls gelegener Asparagin- (N-) oder Glutamin-(Q-)rest zu A, C, F, G, H, I, K, L, M, P, R, S, T, V, W oder Y ausgetauscht wird, wobei es sich bei dem Ausgangsmolekül um folgende α-Amylase handelt: α-Amylase aus *Bacillus sp.* A 7-7 (DSM 12368).

10. Verfahren nach Anspruch 9 zur Erhöhung der Stabilität einer α-Amylase gegenüber einer über das Lösungsmittel bei erhöhten Temperaturen oder hohen pH-Werten ausgeübten Hydrolyse.

11. Verfahren nach Anspruch 9 oder 10, wobei das N durch ein A, G, K, R, S oder T, vorzugsweise durch ein S oder T beziehungsweise das Q durch ein A, G, I, K, R, S oder T, vorzugsweise durch ein S oder T ersetzt wird.

12. Verfahren nach einem der Ansprüche 9 bis 11, wobei der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% (nicht dem Solvens zugänglich) bis 100% (in einem hypothetischen Pentapeptid GGXGG enthalten) berechnet wird.

13. Verfahren nach einem der Ansprüche 9 bis 12, wobei mehrere der genannten Aminosäureaustausche durchgeführt werden, vorzugsweise 2 bis 10, besonders bevorzugt 3 bis 8, ganz besonders bevorzugt 4 bis 6.

14. Verfahren nach einem der Ansprüche 9 bis 13, wobei es sich bei dem Ausgangsmolekül um eine α-Amylase handelt, deren Aminosäuresequenz zu der in SEQ ID NO. 2 angegebenen Aminosäuresequenz in den Positionen +1 bis 485 mindestens zu 96% identisch ist, vorzugsweise zu 98%, besonders bevorzugt zu 100%.

15. Verfahren nach einem der Ansprüche 9 bis 14, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

16. Verfahren nach Anspruch 15, wobei die eingebrachte α-Amylase eine α-Amylase-Variante mit den zusätzlichen Punktmutationen in einer oder mehreren der folgenden Positionen ist: 5, 167, 170, 177, 202, 204, 271, 330, 377, 385 und 445, in der Zählung des maturen Proteins gemäß SEQ ID NO. 2.

17. Verfahren nach Anspruch 16, wobei es sich um folgende Punktmutationen handelt: 5A, 167R, 170P, 177L, 202L, 204V, 271 D, 330D, 377R, 385S und/oder 445Q.

18. Nukleinsäure, codierend für eine α-Amylase-Variante nach einem der Ansprüche 1 bis 8.

19. Nukleinsäure nach Anspruch 18, welche sich von einer Nukleinsäure gemäß SEQ ID NO. 1 ableitet, oder von einer Variante hiervon mit zunehmend bevorzugt 1, 2, 3, 4, 5, 6, 7, 8, 9, 10 oder 11 zusätzlichen Punktmutationen zu solchen Mutationen, die zu einem der mit den Ansprüchen 1 oder 2 bezeichneten Aminosäureaustausche führen.

20. Vektor, enthaltend eine Nukleinsäure nach Anspruch 18 oder 19.

21. Klonierungsvektor nach Anspruch 20.

22. Expressionsvektor nach Anspruch 20.

23. Zelle, die nach gentechnischer Modifizierung eine der in den Ansprüchen 18 oder 19 bezeichneten Nukleinsäuren enthält.

24. Zelle nach Anspruch 23, wobei die genannte Nukleinsäure Teil eines Vektors ist, insbesondere eines Vektors nach Anspruch 21 oder 22.

25. Zelle nach Anspruch 23 oder 24, bei der es sich um ein Bakterium handelt, insbesondere eines, das die gebildete α-Amylase-Variante ins umgebende Medium sekretiert.

26. Zelle nach Anspruch 25, wobei es sich um ein gramnegatives Bakterium handelt, insbesondere eines der Gattungen *Escherichia coli, Klebsiella, Pseudomonas* oder *Xanthomonas,* insbesondere um Stämme von *E. coli* K12, *E. coli* B oder *Klebsiella planticola,* und ganz besonders um Derivate der Stämme *Escherichia coli* BL21 (DE₃), *E. coli* RV308, *E. coli* DH5α, E.*coli* JM109, *E. coli* XL-1 oder *Klebsiella planticola* (Rf).

27. Zelle nach Anspruch 25, wobei es sich um ein grampositives Bakterien handelt, insbesondere eines der Gattungen *Bacillus, Staphylococcus* oder *Corynebacterium,* ganz besonders der Species *Bacillus lentus, B. licheniformis, B. amyloliquefaciens, B. subtilis, B.* globigii oder *B.* alcalophilus, *Staphylococcus camosus* oder *Corynebacterium glutamicum,* und hierunter wiederum ganz besonders bevorzugt um ein Derivat von *B. licheniformis* DSM 13.

28. Verfahren zur Herstellung einer α-Amylase-Variante nach einem der Ansprüche 1 bis 8.

29. Verfahren nach Anspruch 28, unter Verwendung einer Nukleinsäure nach einem der Ansprüche 18 oder 19, besonders bevorzugt unter Verwendung eines Vektors nach einem der Ansprüche 20 bis 22, ganz besonders bevorzugt unter Verwendung einer Zelle nach einem der Ansprüche 23 bis 27.

30. Mittel, enthaltend eine α-Amylase-Variante nach einem der Ansprüche 1 bis 8.

31. Mittel nach Anspruch 30, wobei es sich um ein Wasch- oder Reinigungsmittel handelt.

32. Wasch- oder Reinigungsmittel nach Anspruch 31, enthaltend 0,000001 Gewichts-Prozent bis 5 Gew.-%, insbesondere 0,00001 bis 3 Gew.-% der α-Amylase-Variante.

33. Wasch- oder Reinigungsmittel nach Anspruch 31 oder 32, welches zusätzlich andere Enzyme enthält, insbesondere hydrolytische Enzyme oder Oxidoreduktasen, besonders bevorzugt weitere Amylasen, Proteasen, Lipasen, Cutinasen, Hemicellulasen, Cellulasen, β-Glucanasen, Oxidasen, Peroxidasen, Perhydrolasen und/oder Laccasen.

34. Wasch- oder Reinigungsmittel nach einem der Ansprüche 31 bis 33, wobei die α-Amylase-Variante durch einen der sonstigen Bestandteile des Mittels stabilisiert und/oder in ihrem Beitrag zur Wasch-, beziehungsweise Reinigungsleistung des Mittels gesteigert wird.

35. Wasch- oder Reinigungsmittel nach einem der Ansprüche 31 bis 34, welches insgesamt fest ist, vorzugsweise nach einem Kompaktierungsschritt für mindestens eine der enthaltenen Komponenten, besonders bevorzugt, daß es insgesamt kompaktiert ist.

36. Wasch- oder Reinigungsmittel nach einem der Ansprüche 31 bis 34, welches insgesamt flüssig, gelförmig oder pastös ist, vorzugsweise unter Verkapselung für mindestens eine der enthaltenen Komponenten, besonders bevorzugt unter Verkapselung mindestens eines der enthaltenen Enzyme, ganz besonders bevorzugt unter Verkapselung der α-Amylase-Variante.

37. Verfahren zur Reinigung von Textilien oder von harten Oberflächen, in welchem in wenigstens einem der Verfahrensschritte eine α-Amylase-Variante nach einem der Ansprüche 1 bis 8 aktiv wird.

38. Verfahren nach Anspruch 37, wobei die α-Amylase-Variante über ein Mittel nach einem der Ansprüche 31 bis 36 eingesetzt wird.

39. Verfahren nach Anspruch 37 oder 38, wobei die α-Amylase in dem betreffenden Verfahrensschritt in einer Menge von 0,01 mg bis 400 mg pro entsprechendem Verfahrensschritt eingesetzt wird, vorzugsweise von 0,02 mg bis 300 mg, besonders bevorzugt von 0,03 mg bis 100 mg.

40. Verwendung einer α-Amylase-Variante nach einem der Ansprüche 1 bis 8 zur Reinigung von Textilien oder von harten Oberflächen.

41. Verwendung nach Anspruch 40, wobei die α-Amylase-Variante über ein Mittel nach einem der Ansprüche 31 bis 36 eingesetzt wird.

42. Verwendung nach Anspruch 40 oder 41, wobei pro Anwendung, vorzugsweise pro Anwendung in einer Geschirrspülmaschine oder einer Waschmaschine 0,01 mg bis 400 mg der α-Amylase-Variante eingesetzt werden, bevorzugt 0,02 mg bis 300 mg, besonders bevorzugt 0,03 mg bis 100 mg.

43. Verwendung einer α-Amylase-Variante nach einem der Ansprüche 1 bis 8 zur Behandlung von Rohmaterialien oder Zwischenprodukten in der Textilherstellung, insbesondere zum Entschlichten von Baumwolle.

## Claims

1. An α-amylase variant with at least one amino acid substitution relative to the starting molecule, whereby at least one asparagine (N) or glutamine (Q) residue located on the surface of the molecule has been changed to A, C, F, G, H, I, K, L, M, P, R, S, T, V, W or Y, wherein the starting molecule is α-amylase from *Bacillus sp.* A 7-7 (DSM 12368).

2. An α-amylase variant according to claim 1, wherein the N has been replaced by an A, G, K, R, S or T, preferably by an S or T, and the Q has been replaced by an A, G, I, K, R, S or T, preferably by an S or T.

3. An α-amylase variant according to either of claims 1 or 2, in which, prior to the amino acid substitution, the stated amino acid residue has an accessibility of at least 10%, preferably of at least 20%, particularly preferably of at least 30%, wherein the accessibility of the amino acid residue in question is calculated on a scale from 0% (not accessible to the solvent) to 100% (present in a hypothetical pentapeptide GGXGG).

4. An α-amylase variant according to any one of claims 1 to 3, wherein a plurality, preferably 2 to 10, particularly preferably 3 to 8, very particularly preferably 4 to 6, of the stated amino acid substitutions have been carried out.

5. An α-amylase variant according to any one of claims 1 to 4, wherein the starting molecule is an α-amylase, the amino acid sequence of which, in positions +1 to 484, is at least 96%, preferably 98%, particularly preferably 100%, identical to the amino acid sequence stated in SEQ ID NO. 2.

6. An α-amylase variant according to any one of claims 1 to 5, wherein the starting molecule is an α-amylase variant with increasingly preferably 1, 2, 3, 4, 5, 6, 7, 8, 9, 10 or 11 additional point mutations.

7. An α-amylase variant according to claim 6 with the additional point mutations in one or more of the following positions: 5, 167, 170, 177, 202, 204, 271, 330, 377, 385 and 445, in the numbering of the mature protein according to SEQ ID NO. 2.

8. An α-amylase variant according to claim 7, wherein the point mutations are as follows: 5A, 167R, 170P, 177L, 202L, 204V, 271 D, 330D, 377R, 385S and/or 445Q.

9. A method for increasing the stability of an α-amylase to hydrolysis effected by solvent, whereby at least one asparagine (N) or glutamine (Q) residue located on the surface of the molecule is changed to A, C, F, G, H, I, K, L, M, P, R, S, T, V, W or Y, wherein the starting molecule is α-amylase from *Bacillus sp.* A 7-7 (DSM 12368).

10. A method according to claim 9 for increasing the stability of an α-amylase to hydrolysis effected by solvent at elevated temperatures or elevated pH values.

11. A method according to claim 9 or 10, wherein the N is replaced by an A, G, K, R, S or T, preferably by an S or T, and the Q has been replaced by an A, G, I, K, R, S or T, preferably by an S or T.

12. A method according to any one of claims 9 to 11, wherein, prior to the amino acid substitution, the stated amino acid residue has an accessibility of at least 10%, preferably of at least 20%, particularly preferably of at least 30%, wherein the accessibility of the amino acid residue in question is calculated on a scale from 0% (not accessible to the solvent) to 100% (present in a hypothetical pentapeptide GGXGG).

13. A method according to any one of claims 9 to 12, wherein a plurality, preferably 2 to 10, particularly preferably 3 to 8, very particularly preferably 4 to 6, of the stated amino acid substitutions are carried out.

14. A method according to any one of claims 9 to 13, wherein the starting molecule is an α-amylase, the amino acid sequence of which, in positions +1 to 485, is at least 96%, preferably 98%, particularly preferably 100%, identical to the amino acid sequence stated in SEQ ID NO. 2.

15. A method according to any one of claims 9 to 14, wherein the starting molecule is an α-amylase variant with increasingly preferably 1, 2, 3, 4, 5, 6, 7, 8, 9, 10 or 11 additional point mutations.

16. A method according to claim 15, wherein the introduced α-amylase is an α-amylase variant with the additional point mutations in one or more of the following positions: 5, 167, 170, 177, 202, 204, 271, 330, 377, 385 and 445, in the numbering of the mature protein according to SEQ ID NO. 2.

17. A method according to claim 16, wherein the point mutations are as follows: 5A, 167R, 170P, 177L, 202L, 204V, 271 D, 330D, 377R, 385S and/or 445Q.

18. A nucleic acid, encoding an α-amylase variant according to any one of claims 1 to 8.

19. A nucleic acid according to claim 18, which is derived from a nucleic acid according to SEQ ID NO. 1 or from a variant thereof with increasingly preferably 1, 2, 3, 4, 5, 6, 7, 8, 9, 10 or 11 additional point mutations to those mutations which lead to an amino acid substitution stated in claims 1 or 2.

20. A vector containing a nucleic acid according to claim 18 or 19.

21. A cloning vector according to claim 20.

22. An expression vector according to claim 20.

23. A cell which, after modification by genetic engineering, contains one of the nucleic acids stated in claim 18 or 19.

24. A cell according to claim 23, wherein the stated nucleic acid is part of a vector, in particular of a vector according to claim 21 or 22.

25. A cell according to claim 23 or 24 which is a bacterium, in particular a bacterium which secretes the α-amylase variant formed into the surrounding medium.

26. A cell according to claim 25, which is a Gram negative bacterium, in particular a bacterium from the genera *Escherichia coli, Klebsiella, Pseudomonas* or *Xanthomonas,* in particular strains of *E. coli* K12, *E. coli* B or *Klebsiella planticola,* and very particularly derivatives of the strains *Escherichia coli* BL21 (DE3), *E. coli* RV308, *E. coli* DH5α, *E. coli* JM109, *E. coli* XL-1 or *Klebsiella planticola* (Rf).

27. A cell according to claim 25, which is a Gram positive bacteria, in particular a bacterium from the genera *Bacillus, Staphylococcus* or *Corynebacterium,* very particularly of the species *Bacillus lentus, B. licheniformis, B. amyloliquefaciens, B. subtilis, B. globigii* or *B. alcalophilus, Staphylococcus carnosus* or *Corynebacterium glutamicum,* and among these in turn very particularly preferably a derivative of *B. licheniformis* DSM 13.

28. A method for producing an α-amylase variant according to any one of claims 1 to 8.

29. A method according to claim 28, using a nucleic acid according to any one of claims 18 or 19, particularly preferably using a vector according to any one of claims 20 to 22, very particularly preferably using a cell according to any one of claims 23 to 27.

30. An agent containing an α-amylase variant according to any one of claims 1 to 8.

31. An agent according to claim 30, wherein it is a washing or cleaning agent.

32. A washing or cleaning agent according to claim 31 containing 0.000001 weight percent to 5 wt.%, in particular 0.00001 to 3 wt.% of the α-amylase variant.

33. A washing or cleaning agent according to claim 31 or 32 which additionally contains other enzymes, in particular hydrolytic enzymes or oxidoreductases, particularly preferably further amylases, proteases, lipases, cutinases, hemicellulases, cellulases, β-glucanases, oxidases, peroxidases, perhydrolases and/or laccases.

34. A washing or cleaning agent according to any one of claims 31 to 33, wherein the α-amylase variant is stabilised by one of the other components of the agent and/or is enhanced in its contribution to the washing or cleaning performance of the agent.

35. A washing or cleaning agent according to any one of claims 31 to 34, which is overall solid, preferably after a compaction step for at least one of the components contained therein, which is particularly preferably overall compacted.

36. A washing or cleaning agent according to any one of claims 31 to 34, which is overall liquid, in gel form or pasty, preferably with encapsulation for at least one of the components contained therein, particularly preferably with encapsulation of at least one of the enzymes contained therein, very particularly preferably with encapsulation of the α-amylase variant.

37. A method for cleaning textiles or hard surfaces, in which an α-amylase variant according to any one of claims 1 to 8 is active in at least one of the method steps.

38. A method according to claim 37, wherein the α-amylase variant is used by means of an agent according to any one of claims 31 to 36.

39. A method according to claim 37 or 38, wherein the α-amylase is used in the method step in question in a quantity of 0.01 mg to 400 mg, preferably of 0.02 mg to 300 mg, particularly preferably of 0.03 mg to 100 mg, per corresponding method step.

40. A use of an α-amylase variant according to any one of claims 1 to 8 for cleaning textiles or hard surfaces.

41. A use according to claim 40, wherein the α-amylase variant is used by means of an agent according to any one of claims 31 to 36.

42. A use according to claim 40 or 41, wherein 0.01 mg to 400 mg, preferably 0.02 mg to 300 mg, particularly preferably 0.03 mg to 100 mg of the α-amylase variant is used per instance of use, preferably per instance of use in a dishwashing machine or a washing machine

43. A use of an α-amylase variant according to any one of claims 1 to 8 for treating raw materials or intermediates in textiles manufacture, in particular for scouring cotton.

## Revendications

1. Variant de l'α-amylase comprenant au moins un échange d'acide aminé par rapport à la molécule de départ, au moins un résidu d'asparagine (N) ou de glutamine (Q) situé à la surface de la molécule ayant été échangé contre un résidu A, C, F, G, H, I, K, L, M, P, R, S, T, V, W, ou Y, dans lequel, en ce qui concerne la molécule de départ, il s'agit de l'α-amylase suivante : l'α-amylase de *Bacillus sp.* A 7-7 (DSM 12368).

2. Variant de l'α-amylase selon la revendication 1, dans lequel le résidu N a été remplacé par un résidu A, G, K, R, S ou T, de préférence par un résidu S ou T, respectivement, le résidu Q a été remplacé par un résidu A, G, I, K, R, S ou T, de préférence par un résidu S ou T.

3. Variant de l'α-amylase selon l'une quelconque des revendications 1 ou 2, dans lequel le résidu d'acide aminé mentionné présente, avant l'échange d'acides aminés, une accessibilité d'au moins 10 %, de préférence d'au moins 20 %, de manière particulièrement préférée d'au moins 30 %, l'accessibilité du résidu d'acide aminé concerné étant calculée sur une échelle allant de 0 % (inaccessible au solvant) à 100 % (contenu dans un pentapeptide hypothétique GGXGG).

4. Variant de l'α-amylase selon l'une quelconque des revendications 1 à 3, dans lequel on a procédé à plusieurs échanges d'acides aminés mentionnés, de préférence à un nombre de 2 à 10, de manière particulièrement préférée de 3 à 8, de manière tout particulièrement préférée de 4 à 6.

5. Variant de l'α-amylase selon l'une quelconque des revendications 1 à 4, dans lequel, en ce qui concerne la molécule de départ, il s'agit d'une α-amylase dont la séquence d'acides aminés manifeste une identité par rapport à la séquence d'acides aminés indiquée dans la SEQ ID NO: 2, aux positions +1 à 484, d'au moins 96 %, de préférence de 98 %, de manière particulièrement préférée de 100 %.

6. Variant de l'α-amylase selon l'une quelconque des revendications 1 à 5, dans lequel, en ce qui concerne la molécule de départ, il s'agit d'un variant de l'α-amylase comprenant les mutations ponctuelles supplémentaires de plus en plus préférées 1, 2, 3, 4, 5, 6, 7, 8, 9, 10 ou 11.

7. Variant de l'α-amylase selon la revendication 6, comprenant les mutations ponctuelles supplémentaires dans une ou plusieurs des positions suivantes : 5, 167, 170, 177, 202, 204, 271, 330, 377, 385 et 445 dans la numérotation de la protéine mature selon la SEQ ID NO: 2.

8. Variant de l'α-amylase selon la revendication 7, dans lequel il s'agit des mutations ponctuelles suivantes: 5A, 167R, 170P, 177L, 202L, 204V, 271 D, 330D, 377R, 385S et 445Q.

9. Procédé pour augmenter la stabilité d'une α-amylase par rapport à une hydrolyse effectuée via le solvant, par lequel au moins un résidu d'asparagine (N) ou de glutamine (Q) situé à la surface de la molécule est échangé contre un résidu A, C, F, G, H, I, K, L, M, P, R, S, T, V, W, ou Y, et dans lequel en ce qui concerne la molécule de départ, il s'agit de l'α-amylase suivante : l'α-amylase de *Bacillus sp.* A 7-7 (DSM 12368).

10. Procédé selon la revendication 9, pour augmenter la stabilité d'une α-amylase par rapport à une hydrolyse effectuée via le solvant à des températures élevées ou à des valeurs de pH élevées.

11. Procédé selon la revendication 9 ou 10, dans lequel le résidu N est remplacé par un résidu A, G, K, R, S ou T, de préférence par un résidu S ou T, respectivement, le résidu Q a été remplacé par un résidu A, G, I, K, R, S ou T, de préférence par un résidu S ou T.

12. Procédé selon l'une quelconque des revendications 9 à 11, dans lequel le résidu d'acide aminé mentionné présente, avant l'échange d'acide aminé, une accessibilité d'au moins 10 %, de préférence d'au moins 20 %, de manière particulièrement préférée d'au moins 30 %, l'accessibilité du résidu d'acide aminé concerné étant calculée sur une échelle allant de 0 % (inaccessible au solvant) à 100 % (contenu dans un pentapeptide hypothétique GGXGG).

13. Procédé selon l'une quelconque des revendications 9 à 12, dans lequel on procède à plusieurs échanges d'acides aminés mentionnés, de préférence à un nombre de 2 à 10, de manière particulièrement préférée de 3 à 8, de manière tout particulièrement préférée de 4 à 6.

14. Procédé selon l'une quelconque des revendications 9 à 13, dans lequel, en ce qui concerne la molécule de départ, il s'agit d'une α-amylase dont la séquence d'acides aminés manifeste une identité par rapport à la séquence d'acides aminés indiquée dans la SEQ ID NO: 2, aux positions +1 à 484, d'au moins 96 %, de préférence de 98 %, de manière particulièrement préférée de 100 %.

15. Procédé selon l'une quelconque des revendications 9 à 14, dans lequel, en ce qui concerne la molécule de départ, il s'agit d'un variant de l'α-amylase comprenant les mutations ponctuelles supplémentaires de plus en plus préférées 1, 2, 3, 4, 5, 6, 7, 8, 9, 10 ou 11.

16. Procédé selon la revendication 15, dans lequel l'α-amylase introduite représente un variant d'une α-amylase avec des mutations ponctuelles supplémentaires dans une ou plusieurs des positions suivantes : 5, 167, 170, 177, 202, 204, 271, 330, 377, 385 et 445 dans la numérotation de la protéine mature selon la SEQ ID NO: 2.

17. Procédé selon la revendication 16, dans lequel il s'agit des mutations ponctuelles suivantes : 5A, 167R, 170P, 177L, 202L, 204V, 271 D, 330D, 377R, 385S et/ou 445Q.

18. Acide nucléique, codant pour un variant de l'α-amylase selon l'une quelconque des revendications 1 à 8.

19. Acide nucléique selon la revendication 18, qui dérive d'un acide nucléique selon la SEQ ID NO: 1, ou d'une de ses variantes comprenant les mutations ponctuelles supplémentaires de plus en plus préférées 1, 2, 3, 4, 5, 6, 7, 8, 9, 10 ou 11 par rapport à des mutations qui donnent lieu à un des échanges d'acides aminés repris dans les revendications 1 ou 2.

20. Vecteur comprenant un acide nucléique selon la revendication 18 ou 19.

21. Vecteur de clonage selon la revendication 20.

22. Vecteur d'expression selon la revendication 20.

23. Cellule qui contient, après une modification basée par génie génétique, un des acides nucléiques repris dans la revendication 18 ou 19.

24. Cellule selon la revendication 23, dans laquelle l'acide nucléique mentionné fait partie d'un vecteur, en particulier d'un vecteur selon la revendication 21 ou 22.

25. Cellule selon la revendication 23 ou 24, qui est une bactérie en particulier une bactérie qui sécrète dans le milieu environnant le variant de l'α-amylase obtenu.

26. Cellule selon la revendication 25, qui est une bactérie Gram-négative, en particulier une bactérie des genres *Escherichia coli, Klebsiella, Pseudomonas* ou *Xanthomonas,* en particulier de souches de *E. coli* K12, de *E. coli* B ou de *Klebsiella planticola,* et de manière tout à fait particulière des dérivés des souches *Escherichia coli* BL21 (DE3), *E. coli* RV308, *E. coli* DH5, *E.coli* JM109, *E. coli* XL-1 ou *Klebsiella planticola* (Rf).

27. Cellule selon la revendication 25, qui est une bactérie Gram-positive, en particulier une bactérie des genres *Bacillus, Staphylococcus* ou *Corynebacterium,* de manière tout à fait particulière des espèces *Bacillus lentus, B. licheniformis, B. amyloliquefaciens, B. subtilis, B. globigii* ou B. *alcalophilus, Staphylococcus camosus* ou *Corynebacterium glutamicum,* et parmi celles-ci, à nouveau de manière tout particulièrement préférée d'un dérivé de *B. licheniformis* DSM 13.

28. Procédé pour la préparation d'un variant de l'α-amylase selon l'une quelconque des revendications 1 à 8.

29. Procédé selon la revendication 28, en utilisant un acide nucléique selon l'une quelconque des revendications 18 ou 19, de manière particulièrement préférée en utilisant un vecteur selon l'une quelconque des revendications 20 à 22, de manière tout particulièrement préférée en utilisant une cellule selon l'une quelconque des revendications 23 à 27.

30. Agent contenant un variant de l'α-amylase selon l'une quelconque des revendications 1 à 8.

31. Agent selon la revendication 30, dans lequel il s'agit d'un agent de lavage ou de nettoyage.

32. Agent de lavage ou de nettoyage selon la revendication 31, contenant le variant de l'α-amylase à concurrence de 0,000001 % en poids à 5 % en poids, en particulier de 0,00001 à 3 % en poids.

33. Agent de lavage ou de nettoyage selon la revendication 31 ou 32, qui contient en outre d'autres enzymes, en particulier des enzymes hydrolytiques ou des oxydoréductases, de manière particulièrement préférée d'autres amylases, protéases, lipases, cutinases, hémicellulases, cellulases, β-glucanases, oxydases, peroxydases, perhydrolases et/ou laccases.

34. Agent de lavage ou de nettoyage selon l'une quelconque des revendications 31 à 33, dans lequel le variant de l'α-amylase est stabilisé via un des autres constituants de l'agent et/ou est augmenté en ce qui concerne sa contribution à la puissance de lavage, respectivement à la puissance de nettoyage de l'agent.

35. Agent de lavage ou de nettoyage selon l'une quelconque des revendications 31 à 34, qui est globalement solide, de préférence après une étape de compactage pour au moins un des composants qu'il contient, de manière particulièrement préférée, qui est globalement compacté.

36. Agent de lavage ou de nettoyage selon l'une quelconque des revendications 31 à 34, qui est globalement sous forme liquide, sous forme de gel ou sous forme de pâte, de préférence par encapsulage pour au moins un des composants qu'il contient, de manière particulièrement préférée via un encapsulage d'au moins une des enzymes qu'il contient, de manière tout particulièrement préférée par encapsulage du variant de l'α-amylase.

37. Procédé pour le nettoyage de textiles ou de surfaces dures, dans lequel, dans au moins une des étapes opératoires, un variant de l'α-amylase selon l'une quelconque des revendications 1 à 8, devient actif.

38. Procédé selon la revendication 37, dans lequel le variant de l'α-amylase est mis en oeuvre via un agent selon l'une quelconque des revendications 31 à 36.

39. Procédé selon la revendication 37 ou 38, dans lequel l'α-amylase est mise en oeuvre dans l'étape opératoire concernée en une quantité de 0,01 mg à 400 mg par étape opératoire correspondante, de préférence de 0,02 mg à 300 mg, de manière particulièrement préférée de 0,03 mg à 100 mg.

40. Utilisation d'un variant de l'α-amylase selon l'une quelconque des revendications 1 à 8 pour le nettoyage de textiles ou de surfaces dures.

41. Utilisation selon la revendication 40, dans laquelle le variant de l'α-amylase est mis en oeuvre via un agent selon l'une quelconque des revendications 31 à 36.

42. Utilisation selon la revendication 40 ou 41, dans laquelle, par application, de préférence par application dans un lave-vaisselle ou dans un lave-linge, on met en oeuvre de 0,01 mg à 400 mg du variant de l'α-amylase, de préférence de 0,02 mg à 300 mg, de manière particulièrement préférée de 0,03 mg à 100 mg.

43. Utilisation d'un variant de l'α-amylase selon l'une quelconque des revendications 1 à 8, pour le traitement de matières premières ou de produits intermédiaires dans la fabrication de textiles, en particulier pour le désencollage du coton.
